# EUROPEAN PATENT APPLICATION

(11) **EP 4 588 481 A1**
(43) Date of publication of application: **23.07.2025**
(21) Application number: 23865801.7
(22) Date of filing: 08.09.2023
(51) Int. Cl.: A61K 35/13, A61K 41/00, A61K 39/00, A61P 35/00

(54) **COMPOSITION FOR PREVENTING OR TREATING CANCER, AND METHOD FOR PREVENTING OR TREATING CANCER BY USING SAME, HAVING ABSCOPAL EFFECT**

(30) Priority: 16.09.2022 KR 20220117466; 07.09.2023 KR 20230119122
(71) Applicant: Korea Nuclear Engineering Co., Ltd., Seoul 04782 (KR)
(72) Inventor: LEE, Se Yup, Seoul 06071 (KR)
(74) Representative: Dehns
(86) International application number: PCT/KR2023/013532
(87) International publication number: WO 2024/058504

(57) **Abstract**

The present invention relates to a method and composition for inducing at least one immune stimulus, for treating primary cancer and cancer of cells dispersed in the human body and its metastases, and in particular for inducing and promoting the patient's own immune system to recognize and kill the cancer cells and establish a memory to prevent recurrence of the cancer disease.

## Description

### [Technical Field]

The present invention relates to a composition for preventing or treating cancer having abscopal effect and a method of treating cancer using the same, and more particularly a method of enhancing abscopal effect for the treatment of cancer and tumors and a composition for inducing abscopal effect.

This invention claims priority to Korean Patent Application No. 10-2022-0117466, filed on September 16, 2022, and Korean Patent Application No. 10-2023-0119122, filed on September 07, 2023, the entire contents of the specification and drawings of each of which are incorporated herein by reference.

### [Background Art]

In general, radiation therapy is known to impair immune function. However, as more is learned about the role of T cells in radiation therapy, new aspects of the immune response associated with radiation therapy, such as immunomodulation, are becoming known. As representative effects at the cellular level exhibited after radiation exposure, there are increased activity of natural killer (NK) cells, increased infiltration of T cells (especially CD8 T cells), increased antigen presentation by dendritic cells, increased production of immunostimulatory cytokines, and the like.

The abscopal effect is particularly well known for its immunomodulatory effect, and refers to the anticancer treatment effect that suppresses cancer growth in areas other than the tumor tissue in the area where radiation was locally irradiated, including metastatic tumors in areas where radiation was not irradiated. That is, this is a case where radiation therapy sensitizes tumor cells to make them more responsive to cancer treatment.

According to the abscopal effect, radiation treatment affects the body's immune system, causing it to attack cancer cells not only in specific areas of the body but also throughout the body. The immune effect allows the elimination of cancer cells not only in the area exposed to radiation, but also in other distant parts of the body. Radiation therapy causes dendritic cells to release specific markers outside the cells, send danger signals using HMGB1 or ATP, and enhance CD8+ T cells to induce an anticancer response. Therefore, cancer not only in the area that was irradiated, but also in other areas that were not irradiated is removed during radiation therapy. Recently, it was revealed that ablative radiotherapy (20 Gy single fraction) with high doses increases the activity of CD8+ cytotoxic T cells, thereby controlling not only cancer in the treatment site but also cancer in untreated distant metastatic sites. Recently, it was reported that when a vaccine (Flt3L) that inhibits TLR9 is administered to low-grade lymphoma and cancer at a specific site is irradiated with radiation, cancer at an untreated distant site also showed a reduced response. In addition, when patients with metastatic lung cancer were administered with granulocy macrophage-colony stimulating factor (GM-CSF) together with radiotherapy, a reduced response was observed in some cancer in areas that did not receive radiotherapy.

Meanwhile, MHC antigen-presented on cancer cells causes CD8+ cytotoxic T lymphocytes to secrete interferon gamma, which promotes the production of PD-1 ligand on tumor cells. This PD-1 ligand binds to PD-1 on T cells and worsens the activity of T cells. Therefore, antibodies (e.g., anti-PD-1 antibody or bispecific T cell recruiting antibodies; CD 133 × CD3 Ab) that can suppress this can be used in cancer treatment.

Immune checkpoint inhibitors have been shown to be effective in treating cancer through several research reports. Clinically, ipilimumab, which acts as an immune checkpoint inhibitor of cytotoxic T-Lymphocyte associated antigen-4 (CTLA-4), nivolumab, which acts on programmed death-1 (PD-1), and the like have been reported to increase the survival rate of patients with advanced melanoma. Another anti-PD-1 Ab, pembrolizumab, has also shown efficacy in stomach cancer, nasopharyngeal cancer, lung cancer, etc. However, the effectiveness of immunotherapy was limited to 10-30% as monotherapy.

In summary, several studies are being conducted to discover ways to enhance the abscopal effect. However, a method of being capable of effectively maximizing the abscopal effect has not yet been presented.

### [Disclosure]

### [Technical Problem]

The present invention has been made in view of the above problems, and it was confirmed that when tumor tissue or cancer cells isolated from a cancer patient are treated with radiation irradiation or a cancer immunotherapy drug to suppress the activity of cancer cells or induce their death, and then re-administered to the cancer patient, the body's immune function against cancer can be activated and the effect of anticancer therapy can be further enhanced so that cancer growth, recurrence, metastasis and the like can be suppressed, thus completing the present invention. In particular, it was confirmed that cancer cells that were induced to suppress activity or die as described above can exert an abscopal effect when administered to a cancer patient.

In addition, it was confirmed that when cancer cells that were induced to suppress activity or die as described above are used in combination with radiation therapy and/or a cancer immunotherapy drug, the anticancer effect is further maximized, anticancer immunity is enhanced, and a strong abscopal effect is achieved.

Therefore, it is an object of the present invention to provide a pharmaceutical composition for preventing or treating cancer, comprising tumor tissue or cancer cells isolated from a cancer patient and applied with one or more treatments selected from the group consisting of (a) and (b) below as an active ingredient:
(a) radiation irradiation; and
(b) a cancer immunotherapy drug.

In addition, the present invention provides a kit for preventing or treating cancer, comprising the composition.

In addition, the present invention provides a method of preparing the composition.

More specifically, it is an object of the present invention to provide a composition for preventing or treating cancer and a method for preventing or treating cancer using the same, which are for the invasive or non-invasive treatment of primary cancer and its metastatic cancer in a patient suffering from cancer or tumor, and which can enhance the anticancer effect by radiation therapy, treatment using anticancer drugs, or immunostimulants, or exert an abscopal effect to prevent the recurrence of cancer after treatment.

It is another object of the present invention to provide a composition for preventing or treating cancer and a method for preventing or treating cancer using the same, which can be used as a tumor vaccine to prevent immune evasion by cancer cells by making the immune system recognize cancer cells more effectively. In particular, the composition according to the present invention can exert an abscopal effect to prevent cancer recurrence in patients at risk of recurrence after cancer treatment, and therefore has high utility as a tumor vaccine.

It is still another object of the present invention to provide a composition that can provide memory and systemic immune response to cancer cells, especially to disseminated cells of micrometastatic cancer, through the abscopal effect, and can be used as a vaccine therapy, and is useful for preventing recurrence after general cancer and tumor treatment, and a method for preventing or treating cancer using the composition.

It is still another object of the present invention to provide a composition that can increase the abscopal effect for the treatment of cancer and tumors of a subject, but can suppress the immune system impairment, local or systemic damage and load, unwanted cancer and tumor mutation or resistance increase, etc., such as radiation irradiation, side effects and load of the subject due to anticancer agents, side effects and load of metabolites due to an immunizing agent, and a method for preventing or treating cancer using the composition.

It is still another object of the present invention to lower or delay the possibility of developing cancer by administering the composition according to the present invention to a subject who does not have cancer, thereby causing the immune system to learn and remember cancer and strengthening immunity against cancer. Therefore, the composition according to the present invention can achieve a cancer prevention effect in a subject with a high risk of developing cancer due to genetic and environmental factors.

It is still another object of the present invention to achieve a more potent anticancer effect by combining the composition according to the present invention with other anticancer therapy. For example, when the composition according to the present invention is used in combination with radiation therapy and/or a cancer immunotherapy drug, it can more effectively suppress the growth of primary tumors (or primary tumors) and also suppress the growth of secondary tumors through the abscopal effect, thereby more effectively suppressing cancer metastasis. Furthermore, the combination therapy can enhance anticancer immune function, thereby achieving synergistic anticancer effects.

For this, the present invention provides a method of extracting an appropriate amount of cancer and tumor tissue from a subject outside the subject's body, inducing attenuation or death of the extracted tissue or cancer cells by one or more physical, chemical, and medical methods, such as radiation irradiation or an immunizing agent, and then re-administering it to the subject to induce an abscopal effect in the subject's body.

### [Technical Solution]

The present invention provides a pharmaceutical composition for preventing or treating cancer, comprising tumor tissue or cancer cells isolated from a cancer patient and applied with one or more treatments selected from the group consisting of (a) and (b) below, as an active ingredient:
(a) radiation irradiation; and
(b) a cancer immunotherapy drug.

Here, the cancer comprises primary cancer, secondary tumor, metastatic cancer, and recurrent cancer.

In addition, the present invention provides a kit for preventing or treating cancer, comprising the composition.

In addition, the present invention provides use of the composition for preventing or treating cancer.

In addition, the present invention provides a method for preventing or treating cancer, comprising administering the composition to a subject in need thereof. Preferably, the subject is a subject from which the tumor tissue or the cancer cells are isolated, or an allogeneic subject of the subject. In one embodiment of the present invention, the method for preventing or treating cancer may further comprise a step of performing anticancer therapy on the subject. The anticancer therapy may be two or more types of anticancer therapy (i.e., different types of anticancer therapy). In addition, the anticancer therapy may be performed simultaneously or sequentially with the administration of the composition. There is no limitation on the order of performance.

In addition, the present invention provides the use of the composition for preparing a medicament for treating cancer.

Furthermore, the present invention provides use of the composition combined with anticancer therapy.

Furthermore, the present invention provides the use of the composition for preparing a preparation that can be used in combination with anticancer therapy.

Furthermore, the present invention provides a method for preventing or treating cancer, comprising a step (i) of administering the composition to a subject in need thereof; and a step (ii) of administering a second anticancer therapy to the subject. The second anticancer therapy may be two or more types of anticancer therapy (i.e., different types of anticancer therapy). The steps (i) and (ii) may be performed simultaneously or sequentially, and there is no limitation on the order in which they are performed. In addition, when performing two or more types of anticancer therapy, there is no limitation on the order of performing each anticancer therapy, and it can be performed simultaneously with the administration of the composition, or it can be performed sequentially.

In one embodiment of the present invention, the tumor tissue or the cancer cells may satisfy one or more features selected from the group consisting of i) to iii) below by the treatment, but is not limited thereto:
i) the activity of cancer cells is weakened;
ii) the activity of cancer cells is stopped; and
iii) the cancer cells are killed.

In another embodiment of the present invention, the composition may comprise autologous tumor tissue or cancer cells of the cancer patient and may be administered to the cancer patient, or may be administered to a cancer patient who is allogeneic with the cancer patient, but is not limited thereto.

In another embodiment of the present invention, the composition may be administered to a subject who does not have cancer to prevent cancer, but is not limited thereto. The subject may be a subject who is genetically or environmentally at high risk of developing cancer.

In another embodiment of the present invention, when the treatment is (a) radiation irradiation, the radiation may satisfy one or more features selected from the group consisting of i) and ii) below, but is not limited to:
i) the radiation is one or more selected from the group consisting of γ-rays, x-rays, ultraviolet rays, laser rays, and infrared rays; and
ii) an irradiation dose of the radiation is 1 to 500 Gy.

In still another embodiment of the present invention, the cancer immunotherapy drug may be one or more selected from the group consisting of an immune checkpoint inhibitor, a co-stimulatory molecule agent, a cytokine therapeutic agent, a CAR-T cell therapeutic agent, and an autologous CD8+ T immune cell therapeutic agent, but is not limited thereto.

In still another embodiment of the present invention, the immune checkpoint inhibitor may be one or more selected from the group consisting of a PD-L1 inhibitor, a PD-1 inhibitor, a CTLA-4 inhibitor, a LAG3 inhibitor, a TIM3 inhibitor, a 4-1BB inhibitor, a LAG-3 inhibitor, a B7-H4 inhibitor, a HVEM inhibitor, a TIM4 inhibitor, a GAL9 inhibitor, a VISTA inhibitor, a KIR inhibitor, a TIGIT inhibitor, and a BTLA inhibitor, but is not limited thereto.

In still another embodiment of the present invention, the composition may satisfy one or more features selected from the group consisting of i) to iv) below when administered to a cancer patient, but is not limited thereto:
i) exerts an abscopal effect;
ii) inhibits cancer metastasis;
iii) reduces tumor load; and
iv) inhibits proliferation of cancer cells.

In still another embodiment of the present invention, the composition is intended for single or multiple administrations, but is not limited thereto.

In still another embodiment of the present invention, the composition may comprise two or more tumor tissues or cancer cells, wherein the two or more tumor tissues or cancer cells may be each subjected to the same treatment or different treatments, but is not limited thereto.

In still another embodiment of the present invention, the composition may be a mixture of two or more tumor tissues or cancer cells, but is not limited thereto.

In still another embodiment of the present invention, the composition may be prepared by respectively formulating the two or more tumor tissues or cancer cells and may be administered simultaneously, separately, or sequentially, but is not limited thereto.

In still another embodiment of the present invention, the composition may be used in combination with anticancer therapy, but is not limited thereto.

In still another embodiment of the present invention, the anticancer therapy may be one or more selected from the group consisting of radiation therapy, chemotherapy, targeted anticancer therapy, and cancer immunotherapy, but is not limited thereto.

In still another embodiment of the present invention, the composition may be used in combination with radiation therapy and cancer immunotherapy, but is not limited thereto.

In still another embodiment of the present invention, the composition may be administered simultaneously, separately, or sequentially with the anticancer therapy, but is not limited thereto.

In still another embodiment of the present invention, the targeted anticancer agent may be one or more selected from the group consisting of a tyrosine kinase inhibitor, a PARP inhibitor, an angiogenesis inhibitor, and a CDK4/6 inhibitor, but is not limited thereto.

In still another embodiment of the present invention, the cancer immunotherapy drug may be one or more selected from the group consisting of an immune checkpoint inhibitor, a co-stimulatory molecule agent, a cytokine therapeutic agent, a CAR-T cell therapeutic agent, and an autologous CD8⁺ T immune cell therapeutic agent, but is not limited thereto.

In still another embodiment of the present invention, the immune checkpoint inhibitor may be one or more selected from the group consisting of a PD-L1 inhibitor, a PD-1 inhibitor, a CTLA-4 inhibitor, a LAG3 inhibitor, a TIM3 inhibitor, a 4-1BB inhibitor, a LAG-3 inhibitor, a B7-H4 inhibitor, a HVEM inhibitor, a TIM4 inhibitor, a GAL9 inhibitor, a VISTA inhibitor, a KIR inhibitor, a TIGIT inhibitor, and a BTLA inhibitor, but is not limited thereto.

In still another embodiment of the present invention, the tumor tissue may be milled and diluted or dissolved in a solution, but is not limited thereto.

In still another embodiment of the present invention, the composition may comprise two or more tumor tissues, wherein the two or more tumor tissues may have identical or different milling particle sizes, but is not limited thereto.

In still another embodiment of the present invention, the cancer may be one or more selected from the group consisting of blood cancer and solid cancer, but is not limited thereto.

In still another embodiment of the present invention, the cancer may be one or more selected from the group consisting of breast cancer, colorectal cancer, lung cancer, head and neck cancer, small cell lung cancer, stomach cancer, liver cancer, blood cancer, bone cancer, pancreatic cancer, skin cancer, head cancer, neck cancer, cutaneous melanoma, intraocular melanoma, uterine cancer, ovarian cancer, rectal cancer, anal cancer, colon cancer, fallopian tube carcinoma, endometrial carcinoma, cervical cancer, vaginal cancer, vulvar carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocyte lymphoma, bladder cancer, kidney cancer, ureteral cancer, renal cell carcinoma, renal pelvic carcinoma, CNS tumors, primary CNS lymphoma, spinal cord tumors, brainstem glioma, and pituitary adenoma, but is not limited thereto.

In addition, the present invention provides a method of preparing the composition, the method comprising: (S1) isolating tumor tissue or cancer cells from a cancer patient; and

(S2) applying one or more treatments selected from the group consisting of (a) and (b) below to the isolated tumor tissue or cancer cells,
(a) radiation irradiation; and
(b) a cancer immunotherapy drug.

In one embodiment of the present invention, the method may further comprise milling the tumor tissue and diluting or dissolving it in a solution after the step (S1), but is not limited thereto.

In still another embodiment of the present invention, the composition may comprise autologous tumor tissue or cancer cells of the cancer patient, and may be administered to the cancer patient or to a cancer patient allogeneic with the cancer patient, but is not limited thereto.

In still another embodiment of the present invention, the composition may be administered to a subject who does not have cancer to prevent cancer, but is not limited thereto. The subject may be a subject with a high risk of developing cancer due to genetic or environmental factors.

In addition, the present invention provides an anticancer vaccine composition or a vaccine, comprising tumor tissue or cancer cells isolated from a cancer patient and applied with one or more treatments selected from the group consisting of (a) and (b) below as an active ingredient:
(a) radiation irradiation; and
(b) a cancer immunotherapy drug.

In one embodiment of the present invention, the vaccine composition or the vaccine may be administered to a subject who does not have cancer or who has been cured of cancer to prevent cancer, but is not limited thereto.

In addition, the present invention provides a method for preventing cancer, comprising administering tumor tissue or cancer cells to which one or more of the above treatments have been applied to a subject in need thereof.

In addition, the present invention provides use of tumor tissue or cancer cells, to which one or more of the above treatments have been applied, for preventing cancer.

Further, the present invention provides use of tumor tissue or cancer cells, to which one or more of the above treatments have been applied, for preparing a cancer prevention agent (e.g., a cancer prevention vaccine).

### [Advantageous effects]

When irradiating a patient with radiation to induce the abscopal effect, there is a problem of damage and necrosis of normal tissue due to radiation exposure. There is a risk that radiation exposure to some cancerous tissues may induce cancerous mutations, leading to the development of other forms of cancerous tissues that are resistant to radiation or are transformed. The present invention was devised to solve the problems, and relates to a composition that can suppress the growth, recurrence, metastasis, etc. of cancer by exerting an excellent abscopal effect to strengthen the body's immune function against tumors and further enhancing the anticancer effect of anticancer therapy. The present invention is characterized by extracting cancerous tissues or cancer cells from a cancer patient, inducing attenuation or apoptosis of the cancer cells through radiation irradiation or a cancer immunotherapy drug treatment, and then re-administering them to the cancer patient. The re-administered cancer cells or tumor tissue can enhance the sensitivity of the immune system to tumors, and thus maximize the anticancer effect of anticancer therapy through the abscopal effect, and minimize the risk of damage, necrosis, or mutation of normal tissues. In addition, the present invention has the effect of reducing minimal residual disease of cancer and tumor, increasing the remission of cancer or tumor, extending the remission period, lowering the recurrence rate of cancer or tumor, preventing metastasis, lowering the metastasis rate, or achieving a combination of two or more thereof. Furthermore, when the composition according to the present invention is used in combination with other anticancer therapy, a more powerful tumor growth inhibition effect and abscopal effect can be achieved, and in particular, the level of immune cells and interferon with anticancer activity can be increased to enhance anticancer immune function, thereby obtaining a synergistic anticancer effect.

### [Description of Drawings]

FIG. 1 is a block diagram illustrating the application sequence of the present invention and the expected positive effects.
FIGS. 2A and 2B illustrate the results of observing the radiation-induced sensitization effect through MTT analysis after irradiating cancer cells with high doses of radiation (FIG. 2A, microscopic observation results; FIG. 2B, comparison results of cell survival rates) (C, control group; RT, radiation treatment group).
FIG. 3 illustrates the results of comparing the growth of primary tumors after IVAM and radiation were used alone or in combination in tumor animal models to confirm the anticancer effect of IVAM treatment and radiation therapy combined according to the present invention.
FIG. 4 illustrates the results of comparing the growth of secondary tumors (tumors in the left flank that were not irradiated) after IVAM and radiation were used alone or in combination in a tumor animal model to confirm the abscopal effect of IVAM treatment and radiation therapy combined according to the present invention.
FIGS. 5A and 5B illustrate the results of observing the primary tumor (right hind limb) and secondary tumor (left flank) through IVIS imaging after IVAM and radiation were used alone or in combination in tumor animal models to confirm the anticancer effect of IVAM treatment and radiation therapy combined according to the present invention (FIG. 5A) and quantifying the extent of tumor growth (FIG. 5B).
FIG. 6 illustrates the results of measuring changes in body weight over time in tumor animal models treated with IVAM or radiation or a combination thereof to determine the toxicity of the IVAM treatment and radiation therapy according to the present invention.
FIG. 7 illustrates a schematic diagram of an animal experiment to confirm the combined effect of IVAM treatment, a cancer immunotherapy drug, and radiation therapy according to the present invention.
FIG. 8 illustrates the results of observing the growth of the primary tumor (the tumor of the right hind limb irradiated with radiation) over time after administering IVAM therapy, a cancer immunotherapy drug (a-PD-L1), and radiation therapy (RT) according to the present invention alone or in a double combination or a triple combination to tumor animal models.
FIG. 9 illustrates the results of observing the growth of secondary tumors (tumors in the left flank that were not irradiated) over time after administering IVAM therapy, a cancer immunotherapy drug, and radiation therapy according to the present invention alone or in double combination or triple combination to tumor animal models.
FIGS. 10A to 10C illustrate the results of observing the extent of tumor growth through IVIS imaging after administering IVAM therapy, a cancer immunotherapy drug, and radiation therapy according to the present invention alone or in double combination or triple combination to tumor animal models.
FIG. 11 illustrates the results of observing changes in mouse weight over time while administering IVAM therapy, a cancer immunotherapy drug, and radiation therapy according to the present invention alone or in double combination or triple combination to tumor animal models.
FIGS. 12A and 12B illustrate the results of comparing the extent of lung metastasis by measuring the number of metastatic nodules in lung tissue after administering IVAM therapy, a cancer immunotherapy drug, and radiation therapy according to the present invention alone or in double combination or triple combination to tumor animal models.
FIGS. 13A to 13C illustrate the results of observing the distribution changes of immune cells (CD8+ T cells, Treg and CD8+ effector memory T cells) in the tumor microenvironment of primary tumors obtained from the tumor animal models. FIGS. 13A and 13B represent one drawing as a whole.
FIGS. 14A and 14B illustrate the changes in various immune cell populations observed in the spleen obtained from the tumor animal models. FIGS. 14A and 14B represent one figure as a whole.
FIG. 15 illustrates the results of observing changes in interferon (IFN-β and IFN-γ) levels in serum obtained from the tumor animal model.
FIG. 16 illustrates the schematic diagram of an animal experiment to confirm long-term survival rates and toxicity by combined treatment with IVAM treatment, a cancer immunotherapy drug, and radiation therapy according to the present invention.
FIGS. 17A and 17B illustrate the results of observing the long-term survival rate of mice (FIG. 17A) and changes in body weight of mice over time (FIG. 17B) by combined treatment with IVAM treatment, a cancer immunotherapy drug, and radiation therapy according to the present invention.
FIG. 18 illustrates the schematic diagram of an animal experiment to confirm the anticancer effect and toxicity by multiple IVAM administrations according to the present invention.
FIG. 19 illustrates the results of observing the extent of tumor growth by multiple IVAM administrations according to the present invention.
FIG. 20 illustrates the results of observing the extent of tumor growth through IVIS imaging after multiple IVAM administrations according to the present invention.
FIG. 21 illustrates the results of observing changes in body weight of mice over time after multiple IVAM administrations according to the present invention.

### [Best Mode]

The present invention relates to a composition that can strengthen the body's immune function against tumors through abscopal effect and enhance the anticancer effect of chemotherapy. It was confirmed that when tumor tissue or cancer cells isolated from a cancer patient are specially treated to induce inhibition of activity or death of cancer cells and then re-administered to the cancer patient, the body's immune function against cancer is activated and the anticancer effect of anticancer therapy is significantly increased, so that the growth, recurrence, and metastasis of cancer are suppressed, thus completing the present invention. In particular, it was confirmed that cancer cells induced to be inactive or killed as described above can exhibit an abscopal effect when administered to cancer patients. Furthermore, it was confirmed that when the composition according to the present invention is used in combination with other chemotherapy, a more powerful tumor growth inhibition effect and abscopal effect can be achieved, and in particular, the level of immune cells and interferon with anticancer activity is increased, thereby enhancing anticancer immune function. Therefore, a synergistic anticancer effect can be obtained by combining the composition of the present invention with other anticancer therapy.

Therefore, the main objective of the present invention is to provide a pharmaceutical composition for preventing or treating cancer, including tumor tissue or cancer cells, which are isolated from a cancer patient and applied with one or more treatments selected from the group consisting of (a) and (b) below, as an active ingredient:
(a) radiation irradiation; and
(b) a cancer immunotherapy drug.

In this specification, tumor tissue or cancer cells to which one or more of the above treatments have been applied; or a pharmaceutical composition including the same may be referred to as an *In Vitro* Abscopal Method (IVAM).

In addition, the composition can not only achieve the effect of preventing or treating cancer by itself by strengthening the body's immune function against cancer, but also further enhance the anticancer effect of known anticancer therapy. Therefore, the composition of the present invention may be used for purposes of preventing or treating cancer, as well as suppressing the appearance or progression of metastatic cancer or recurrent cancer. For example, the composition according to the present invention may be used as a vaccine composition for preventing cancer, or may be utilized to manufacture a vaccine for preventing cancer.

Conventionally, when irradiating patients with radiation for abscopal effect, there was an unavoidable problem of damage and necrosis of normal tissue due to radiation exposure. In particular, radiation exposure to some cancerous tissues may cause cancerous mutations, which may lead to the development of other forms of cancerous tissue that are resistant to radiation or are transformed. However, the composition of the present invention has the advantage of preventing problems such as damage, necrosis, and mutation of normal tissues by extracting cancerous tissues from a cancer patient and applying physical, chemical, and/or medical treatment, such as radiation irradiation, to the extracted cancerous tissues outside the body to attenuate or kill cancer cells or tumor tissues. As described above, when tumor tissue or cancer cells that have been weakened or killed through radiation irradiation are re-administered to a cancer patient, the body's immune system recognizes, attacks, and/or preys on the cancer cells that have been killed or have reduced activity by heat treatment, and through the experience of attacking the killed cancer cells, the body's sensitivity to other cancer cells increases, allowing it to recognize, attack, and kill them more sensitively. That is, according to the present invention, an abscopal effect may be expected through tumor tissue or cancer cells to which radiation irradiation has been applied. In particular, the present inventors confirmed that when cancer cells irradiated in vitro as described above were re-administered to a tumor animal model and then used in combination with radiation therapy, not only the anticancer effect of radiation therapy was further increased, but also metastatic cancer was also effectively suppressed.

In addition, the present invention is characterized by treating cancerous tissues extracted from a cancer patient with a cancer immunotherapy drug to induce death or attenuation of cancer cells or tumor tissues in vitro, and then re-administering the same to the cancer patient. The immune system of a cancer patient easily recognizes, attacks, and/or preys on the re-administered tumor tissue or cancer cells, and through the experience of attacking the killed cancer cells, it becomes more sensitive to other cancer cells in the body, so it can recognize, attack, and kill them more sensitively. That is, according to the present invention, an abscopal effect may be expected through tumor tissue or cancer cells treated with the cancer immunotherapy drug.

Tumor tissue or cancer cells that have been specially treated as described above stimulate the immune system and induce the same effect as an immunostimulant when re-administered to the cancer patient from which they originated. That is, if tumor tissue or cancer cells are reintroduced into the body in a weakened or killed state through special treatment even if they were not previously recognized by the immune system, the immune system can attack them and obtain a learning effect that allows it to recognize cancer cells and metastatic cells that were not previously recognized. Therefore, the immune system of a subject that has acquired the ability to recognize cancer cells through learning can attack malignant cells throughout the body.

That is, since the administration of the vaccinated tissue cells that induce the abscorpal of the present invention triggers an immune response and activates the immune system, the administration of the vaccinated tissue cells of the present invention is useful for the treatment of primary cancer and its metastases and for preventing the recurrence of cancer disease in patients with a weak or suppressed immune system.

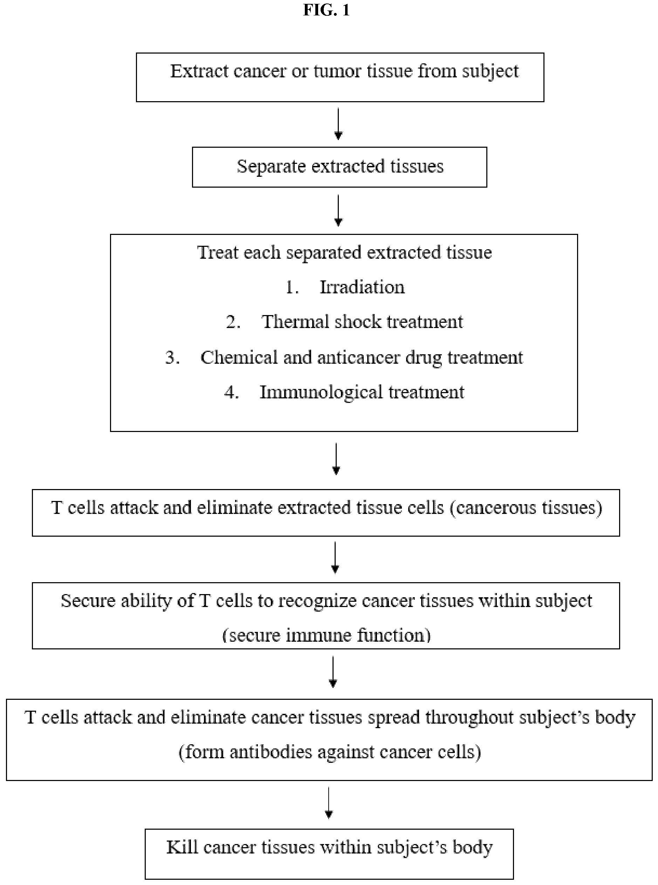

The order of applying the composition according to the present invention to a subject and the abscopal effect and anticancer effect thereof are shown schematically in FIG. 1.

The term "cancer" as used herein refers to a disease characterized by uncontrolled cell growth. Due to such abnormal cell growth, a mass of cells called a tumor is formed, infiltrates surrounding tissues, and in severe cases, metastasizes to other organs of the body. Academically, it is also called a neoplasm. Cancer is an incurable chronic disease that, in many cases, cannot be fundamentally cured even with treatment with surgery, radiation, and chemotherapy, causing pain to patients and ultimately leading to death. There are various causes of cancer, but they are divided into internal and external factors. The exact mechanism by which normal cells are transformed into cancer cells has not been clearly identified, but it is known that a significant number of cancers are caused by external factors such as environmental factors. Internal factors include genetic factors, immunological factors, and the like, while external factors include chemicals, radiation, viruses, and the like. Genes involved in the development of cancer include oncogenes and tumor suppressor genes, and cancer occurs when the balance between the genes is disrupted by the internal or external factors described above. In the present invention, cancer includes all of primary cancer, radiation-treated cancer, non-radiation-treated cancer, metastatic cancer, and recurrent cancer. In addition, the composition according to the present invention may be particularly suitable for the treatment of non-immunogenic tumors or cancers.

In addition, in the present invention, cancer may include all types of solid tumors and all types of blood cancer. As a non-limiting example, the cancer of the present invention may be selected from the group consisting of adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, carcinoma of unknown primary (CUP)- syndrome, colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the otolaryngeal region), colon carcinoma, craniopharyngiomas, oral cancer (cancer of the oral area and lips), cancer of the central nervous system, liver cancer, liver metastases, leukemia, eyelid tumor, lung cancer, lymph node cancer (Hodgkin's/NonHodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycobacterium tuberculosis), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, and tongue cancer. For example, it may be selected from the group consisting of astrocytomas, glioblastomas, and renal cell carcinomas.

The present invention may also be seen as inducing vaccination by applying radiation irradiation and/or immunological treatment to isolated tumor tissue or cancer cells of a cancer patient. Therefore, in one embodiment of the present invention, a vaccine composition for cancer prevention including the tumor tissue or cancer cells to which the treatment has been applied can be manufactured, and the vaccine composition can enhance the function of a cancer patient's immune system to recognize cancer cells that it did not recognize as targets for removal because it judged them as its own body tissues (i.e., increased sensitivity to cancer). In addition, the vaccine composition enables the immune system to recognize metastatic cancer when it occurs, and to create a memory for the cancer so that it can recognize new cancer cells that recur even after successful cancer treatment in a patient. The composition of the present invention comprehensively exerts the above effects, resulting in personalized tumor and cancer vaccination in the subject's body. In other words, the composition of the present invention acts as an immunostimulant, enhancing the subject's immune system's ability to recognize and eliminate cancer cells, eradicate metastatic cancer, and recognize and eliminate new cancer cells that recur after successful cancer treatment.

In one embodiment of the present invention, the tumor tissue or cancer cells may be tumor tissue or cancer cells isolated from an individual in need of the prevention or treatment of cancer. That is, the tumor tissue or cancer cells may be autologous tumor tissue or cancer cells of a subject in need of cancer prevention or treatment. In another embodiment of the present invention, the tumor tissue or cancer cells may be tumor tissue or cancer cells isolated from a subject different from the subject in need of prevention or treatment of cancer. That is, the tumor tissue or cancer cells may be allogeneic tumor tissue or cancer cells of a subject in need of cancer prevention or treatment. Therefore, the pharmaceutical composition according to the present invention may be administered to a cancer patient from whom the tumor tissue or cancer cells included as an active ingredient are isolated (i.e., re-administering killed or attenuated autologous tumor tissue or cancer cells), or may be administered to a subject different from the cancer patient from whom the tumor tissue or cancer cells are isolated (i.e., administering killed or attenuated allogeneic tumor tissue or cancer cells).

In particular, the composition according to the present invention may be administered to a subject who does not have cancer (i.e., a normal subject) to prevent cancer. For example, the composition according to the present invention may be characterized by containing, as an active ingredient, tumor tissue or cancer cells isolated from a cancer patient and attenuated or killed by treatment according to the present invention, and being administered to a subject being the same allogeneic as the cancer patient, wherein the subject may be a subject that does not have cancer. Therefore, even if a subject is likely to develop cancer due to genetic or environmental reasons, the immune system can be strengthened against cancer by receiving attenuated or killed allogeneic tumor tissue or cancerous tissue through treatment according to the present invention so that the immune system experience and learn about cancer, thereby preventing or delaying the occurrence of cancer.

The vaccinated tumor tissue or cancer cells of the present invention may act throughout the body through the abscopal effect, etc. That is, the present invention enables systemic treatment by only extracting local cancerous tissues. The composition according to the present invention can act throughout the body, so it can discover tumor antigens that were not discovered by existing anticancer drugs or radiation therapy, and treat cancer without artificial non-invasive or invasive measures that put a burden on the body. In addition, this immune-inducing function may be induced even in weak patients without any separate physical burden.

The term "abscopal effect" as used herein refers to a systemic anticancer effect that suppresses metastatic tumors in areas not directly irradiated with radiation, in addition to the local area directly irradiated with radiation. Therefore, the abscopal effect causes the shrinkage of tumors distributed in other parts of the body beyond the local tumor treatment range. However, the abscopal effect cannot be said to be induced only by radiation irradiation, and may also be induced by treatment with immunotherapy, etc.

In the present invention, substances that induce the abscopal effect, i.e., substances that induce antibodies specific to cancer cells, such as tumor antigens, are considered part of the vaccine in a broad sense. Therefore, the abscopal effect-inducing substance may be expressed as an "abscopal effect-inducing vaccine."

In particular, the present invention plays an important role as an immunostimulant useful in the treatment of primary cancer and its metastatic cancer in scattered cell states that cannot be detected by imaging methods, and is useful in the prevention of recurrence of cancer diseases. This immunostimulatory method causes a systemic vaccine effect that activates the immune system, which is particularly useful in the treatment of metastatic cancer and primary cancer that cannot be detected by prior art methods, and is particularly useful in preventing the recurrence of successfully treated cancer.

By the present invention, the treatment of the following primary cancer types may be achieved, metastatic cancers of the following cancer types may be treated, and the recurrence of the following cancer types may also be prevented:

adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, carcinoma of unknown primary (CUP)- syndrome, colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the otolaryngeal region), colon carcinoma, craniopharyngiomas, oral cancer (cancer of the oral area and lips), cancer of the central nervous system, liver cancer, liver metastases, leukemia, eyelid tumor, lung cancer, lymph node cancer (Hodgkin's/NonHodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycobacterium tuberculosis), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, and tongue cancer. For example, it may be particularly suitable for the treatment of astrocytomas, glioblastomas, pancreatic cancer, bronchial cancer, breast cancer, colorectal cancer, ovarian cancer, stomach cancer, laryngeal cancer, malignant melanoma, esophageal cancer, cervical cancer, liver cancer, bladder cancer, and renal cell carcinoma.

In other words, the composition according to the present invention enables the patient's immune system to recognize that cancer cells of the above-mentioned cancer types are different from the subject's normal cells and to kill them.

Furthermore, the composition according to the present invention is applied by only performing radiation irradiation, anticancer treatment, etc. outside the body on tumor tissue or cancer cells isolated from a cancer patient, and the treatments are not directly applied to a cancer patient, so there is no concern that serious side effects will occur due to the treatments. For example, tissues or cancer cells extracted from a patient are completely killed or are injected into the patient's body in a weakened state that prevents them from growing and proliferating, so they cannot exist as other mutant cancer cells. Therefore, the application of the present invention is suitable for combination with standard treatment involving one or more of the standard treatments developed so far (surgical operation, radiation therapy, anticancer treatment), and general cancer treatment methods such as immunological treatment.

In the present invention, the treatment may be radiation irradiation. In the present invention, radiation may be selected from, but is not limited to, α-rays, β-rays, y-rays, x-rays, UV, infrared, near-infrared, hadron rays, deuteron rays and the like.

When radiation is applied to tumor tissue or cancer cells extracted from a cancer patient, the following mechanisms may be expected: Normal cancer cells hide as much as possible the factors that can be recognized by the immune system, but when irradiated with radiation, various MHC antigens are expressed at high levels. Cancer cells killed by radiation irradiation produce large amounts of factors such as HMGB-1 or ATP, which activate the innate immune system. For example, cancer cells irradiated with radiation have higher levels of ICAM-1 expression on their surface, making it easier for immune cells to recognize and attack them. In this way, cancer cells that are killed or dying due to radiation not only present a clear marker (Fas receptor) such that immune cells can better recognize them (Calreticulin) and kill them, but also send more combat signals, i.e., activation signals (NKG2D), that arm the immune cells. In addition, radiation-irradiated cancer cells produce more chemokines that recruit immune cells. Therefore, when a composition containing tumor tissue or cancer cells irradiated with radiation according to the present invention is administered to a cancer patient, immune cells can gather around the administered tumor tissue or cancer cells, and thus the possibility of recognition of cancer cells by immune cells increases. Therefore, the vaccine effect induced by the present invention contributes to the activation of the immune system in the subject's body. Furthermore, since the radiation irradiation of the present invention is performed in vitro, unlike existing methods, as described above, it can block the possibility of mutation of cancerous tissues in the subject's body (in-vivo) due to radiation.

In the present invention, the dose of the irradiated radiation may be 1 to 500 Gy, 1 to 400 Gy, 1 to 300 Gy, 1 to 200 Gy, 1 to 100 Gy, 1 to 80 Gy, 1 to 50 Gy, 1 to 30 Gy, 1 to 10 Gy, 5 to 10 Gy, 10 to 200 Gy, 10 to 100 Gy, 10 to 80 Gy, 20 to 60 Gy, 30 to 60 Gy, or 40 to 60 Gy, but is not limited thereto.

The radiation may be administered in single or multiple doses. For example, multiple-dose equivalents may be irradiated instead of irradiating a single dose of radiation.

In the present invention, the treatment may be a cancer immunotherapy drug.

The term "anticancer agent" used in this specification collectively refers to substances used to treat malignant tumors. Most anticancer drugs are drugs that interfere with various metabolic pathways of cancer cells, mainly inhibiting the synthesis of nucleic acids or exhibiting anticancer activity. Anticancer drugs currently used for cancer treatment are classified into six categories based on their biochemical mechanisms of action: alkylating agents, antimetabolites, antibiotics, mitotic inhibitors (vinca alkaloids), hormones, and others. However, anticancer drugs according to the present invention may not be included in the categories.

"Cancer immunotherapy" using a cancer immunotherapy drug is a cancer treatment that activates the body's immune system to fight cancer cells. A cancer immunotherapy drug exhibits an anticancer effect by enhancing the specificity, memory, and adaptiveness of the immune system. That is, it precisely attacks only cancer cells by utilizing the human body's immune system, has fewer side effects, and utilizes the memory and adaptive abilities of the immune system, so patients who respond to a cancer immunotherapy drug can see a continuous anticancer effect. Preferably, the cancer immunotherapy drug may be one or more selected from the group consisting of an immune checkpoint inhibitor, a co-stimulatory molecule agent, a cytokine therapeutic agent, a CAR-T cell therapeutic agent, and an autologous CD8+ T immune cell therapeutic agent, but is not limited thereto. An immune checkpoint inhibitor refers to an agent that inhibits an immune checkpoint involved in the immune evasion mechanism of cancer cells. Some cancer cells evade immunity by utilizing immune checkpoints of immune cells. An immune checkpoint inhibitor binds to the binding site of cancer cells and T cells, blocking immune evasion signals and preventing the formation of immunological synapses. As a result, T cells that are not immune evasion-blocked have a mechanism to destroy cancer cells. The immune checkpoint inhibitor may be at least one selected from the group consisting of a PD-L1 inhibitor, a PD-1 inhibitor, a CTLA-4 inhibitor, a LAG3 inhibitor, a TIM3 inhibitor, a 4-1BB inhibitor, a LAG-3 inhibitor, a B7-H4 inhibitor, an HVEM inhibitor, a TIM4 inhibitor, a GAL9 inhibitor, a VISTA inhibitor, a KIR inhibitor, a TIGIT inhibitor, and a BTLA inhibitor, but is not limited thereto. The inhibitor is not limited to a specific type and includes, without limitation, anything that can inhibit the function or expression of the target (immune checkpoint protein). As specific examples, there are antibodies or fragments thereof (Fab, Fab', F(ab')2, scFv, (scFv)2, Fv, dsFv, diabody, nanobody, Fd, and Fd', etc.), compounds, other peptides, and the like. Examples of commercialized immune checkpoint inhibitors include, but are not limited to, pembrolizumab (Keytruda), ipilimumab (Yervoy), nivolumab (Opdivo), atezolizumab (Tecentriq), cemiplimab (Libtayo), atezolizumab (Tecentriq), avelumab (Bavencio), durvalumab (Imfinzi), tremelimumab (Imjuno), relatlimab, nivolumab, and the like.

Immunotherapy may include immune-cell therapy in addition to a cancer immunotherapy drug. Genetic modification of immune cells is well known as an immuno-cellular therapy for cancer. These immune-cell therapies are based on manipulating autologous or allogeneic immune cells and administering them to a subject in need. Immune-cell-based therapies include natural killer cell therapy, dendritic cell therapy, and T-cell immunotherapy including naive T-cells, effector T-cells (also known as T-helper cells), cytotoxic T-cells, regulatory T-cells (Tregs) and the like.

The genetically modified immune cells may be T-cells. In another embodiment, the T-cells are naive T-cells. In another embodiment, the T-cells are naive CD4+ T-cells. In another embodiment, the T-cells are naive T-cells. In another embodiment, the T-cells are naive CD8+ T-cells. In another embodiment, the genetically modified immune cells are natural killer (NK) cells. In another embodiment, the genetically modified immune cells are dendritic cells. In another embodiment, the genetically modified T cells are cytotoxic T lymphocytes (CTL cells). In another embodiment, the genetically modified T cells are regulatory T cells (Treg). In another embodiment, the genetically modified T cells are chimeric antigen receptor (CAR) T cells. In another embodiment, the genetically modified T cells are genetically modified T-cell receptor (TCR) cells.

Immunotherapy involves cytokines. Cytokines are granulocyte-macrophage colony-stimulating factors (GM-CSF), interleukins such as IL-2, and/or interferons such as IFN-alpha. Additional approaches to enhance tumor-targeted immune responses include additional immune checkpoint inhibition. Immune checkpoint inhibition includes anti-CTLA4, anti-PD-1, anti-PD-L1, anti-PD-L2, anti-TIM-3, anti-LAG-3, anti-A2aR or anti-KIR antibodies. Immunotherapy includes co-stimulatory receptor agonists such as anti-OX40 antibodies, anti-GITR antibodies, anti-CD137 antibodies, anti-CD40 antibodies and anti-CD27 antibodies. Immunotherapy involves the suppression of T regulatory cells (T regulation), myeloid-derived suppressor cells (MDSC) and cancer-associated fibroblasts (CAF). Immunotherapy involves the stimulation of innate immune cells such as natural killer (NK) cells, macrophages and dendritic cells. Additional immune-stimulating therapies include IDO inhibitors, TGF-beta inhibitors, IL-10 inhibitors, stimulators of interferon genes (STING) agonists, toll-like receptor (TLR) agonists (e.g., TLR7, TLR8 or TLR9), tumor vaccines (e.g., whole tumor cell vaccines, peptide and recombinant tumor-associated antigen vaccines), adoptive cell therapy (ACT) (e.g., T cells, natural killer cells, TIL and LAK cells), and ACT with genetically engineered receptors (e.g., chimeric antigen receptors (CARs) and T cell receptors (TCRs)). Combinations of these agents, such as combinations of immune checkpoint inhibitors, checkpoint inhibition + T-cell co-stimulatory receptor agonist action, and checkpoint inhibition + TIL ACT, may be used. Additional anticancer treatments include combinations of immune checkpoint inhibitors (e.g., avelumab), 4-1BB (CD-137) agonists (e.g., utomirumab) and OX40 (TNFRS4) agonists.

The composition according to the present invention may include only one type of tumor cells or cancer cells that have been specifically treated as an active ingredient, or may contain two or more types of tumor tissues or cancer cells. Here, the two or more tumor tissues or cancer cells may have been subjected to the same type of treatment, but may have been subjected to different types of treatment.

When the composition according to the present invention includes two or more tumor tissues or cancer cells, the composition may be in the form of a mixture in which the two or more tumor tissues or cancer cells are mixed. That is, the two or more tumor tissues or cancer cells may be administered simultaneously.

The composition may be in a form in which the two or more tumor tissues or cancer cells are each formulated and administered simultaneously or sequentially. In this case, the composition may be a pharmaceutical composition for combination administration for the simultaneous or sequential administration of the tumor tissues or the cancer cells. In the case of sequential administration, there is no restriction on the order of administration, and the administration regimen may be appropriately adjusted depending on the patient's condition, etc.

In addition, the composition according to the present invention may be used in combination with other chemotherapy. The composition according to the present invention may maximize the anticancer effect of anticancer drugs by increasing the sensitivity of the subject to chemotherapy through the abscopal effect and regulating the activity and level of immune cells and immune regulatory factors involved in the immune response. Therefore, the composition of the present invention may enhance the anticancer effect of combined anticancer therapy and reduce side effects. In addition, it was confirmed that, when the composition according to the present invention was used in combination with radiation therapy and a cancer immunotherapy drug, tumor growth was suppressed more effectively, the abscopal effect by radiation therapy was further increased, and cancer metastasis was reduced. In addition, the excellent anticancer effect of combination therapy was confirmed to be the result of an increase in anticancer immune cells in the tumor and spleen and an increase in the level of anticancer interferon in the serum. In addition, it was confirmed that the composition according to the present invention maintained the survival rate without causing side effects such as toxicity even for a long period of 50 days when used in combination with anticancer therapy.

According to an embodiment of the present invention, the composition according to the present invention was confirmed to maintain survival rate without showing toxicity when administered once a day or 1 to 3 times, 1 to 2 times, or 2 to 3 times at intervals of 3 to 4 days. Accordingly, the composition according to the present invention may be administered 1 to 3 times, but is not limited thereto.

The term "combination" as used herein can be achieved by administering (treating) the individual components of the treatment regimen simultaneously, sequentially, or separately. The combination therapy effect is obtained by performing two or more anticancer therapy treatments simultaneously, sequentially, or alternately at regular or indefinite intervals. Combination therapy can be defined as a treatment that provides a synergistic effect while being therapeutically superior to the efficacy that can be obtained by administering one or the other of the components of the combination therapy at regular doses, for example, but not limited to, as measured by the degree of response, the rate of response, the time to disease progression, or a survival time.

The composition according to the present invention may be used in combination with, but is not limited to, one or more selected from the group consisting of radiation therapy, chemotherapy, targeted anticancer therapy, and cancer immunotherapy.

In the present invention, "targeted anticancer agent" refers to an agent that exhibits an anticancer effect by targeting proteins or genes that are specifically changed in cancer cells or cancer tissues and interfering with molecular activities involved in the growth and occurrence of cancer. The targeted anticancer agent may be one or more selected from the group consisting of tyrosine kinase inhibitors (TKI), poly-ADP ribose polymerase inhibitors (PARP inhibitors), angiogenesis inhibitors, cyclin-dependent kinases 4/6 inhibitors (CDK4/6 inhibitors), hormonal therapy drugs, and antibody-drug conjugates, but is not limited thereto.

The term "chemotherapeutic agent" as used in this specification refers to a first-generation anticancer agent, also called "cytotoxic anticancer agent" or "chemical anticancer agent".

The composition according to the present invention may be characterized by being used in combination with radiation therapy and cancer immunotherapy. Detailed descriptions of the cancer immunotherapy drug and the cancer immunotherapyare omitted as they have been described above.

The composition according to the present invention may be administered simultaneously, separately, or sequentially with the chemotherapy. In addition, each chemotherapy may be performed repeatedly.

For example, the composition according to the present invention may be administered 1 to 3 days, 1 to 5 days, 1 to 7 days, 1 to 10 days, 1 to 20 days, 1 to 30 days, 5 to 15 days, 5 to 20 days, 10 minutes to 2 hours, 10 minutes to 1 hour, 10 minutes to 50 minutes, or 10 minutes to 30 minutes before performing the anticancer therapy, may be administered simultaneously with the anticancer therapy, and may be administered 5 minutes to 10 minutes, 30 minutes to 1 hour, 6 hours to 12 hours, 12 hours to 24 hours, 1 day to 3 days, 1 day to 5 days, 1 day to 7 days, 1 day to 10 days, 1 day to 20 days, 1 day to 30 days, 5 days to It may be administered 15 days later, or 5 to 20 days after performing the anticancer therapy. In addition, the composition according to the present invention may be administered before or after performing the anticancer therapy.

The anticancer therapyis not limited and may include any anticancer therapyknown in the art. As non-limiting examples, there are surgical therapy, chemotherapy (e.g., administration of protein kinase inhibitors or EGFR-targeted therapeutics), embolization therapy, chemoembolization therapy, radiation therapy, cryotherapy, hyperthermia, phototherapy, radiation ablation, hormonal therapy, immunotherapy, small molecule therapy, receptor kinase inhibitor therapy, anti-angiogenic therapy, cytokine therapy or biological therapy, monoclonal antibody, siRNA, miRNA, antisense oligonucleotide, ribozyme or gene therapy, and the like.

In treatment with anticancer drugs, actinomycin D, aminoglutethimide, amsacrin, anastrozol, antagonists of purine and pyrimidine bases, anthracycline, aromatase inhibitors, asparaginase, antiestrogenes, bexaroten, bleomycin, buselerin, busulfan, camptothecin derivates, capecitabin, carboplatin, carmustine, chlorambucil, cladribin, cyclophosphamide, cytarabin, cytosinarabinoside, alkylating cytostatics, dacarbacin, dactinomycin, daunorubicin, docetaxel, doxorubicin (adriamycin), doxorubicin lipo (Lipo-Dox), epirubicin, estramustine, etoposid, exemestan, fludarabin, fluorouracil, folic acid antagonists, formestan, gemcitabin, glucocorticoides, goselerin, hormone antagonists, hycamtin, hydroxy urea, idarubicin, ifosfamid, imatinib, irinotecan, letrozol, leuprorelin, lomustin, melphalan, mercaptopurine, methotrexate, miltefosin, mitomycine, mitosis inhibitors, mitoxantron, nimustine, oxaliplatin, paclitaxel, pentostatin, procarbacin, tamoxifen, temozolomid, teniposid, testolacton, thiotepa, thioguanine, topoisomerase inhibitors, topotecan, treosulfan, tretinoin, triptorelin, trofosfamide, vinblastine, vincristine, vindesine, vinorelbine, antibiotics with cytotoxic activity, and the like may be used.

Treatment with an immunizing agent typically relies on the use of immune effector cells and molecules to target and destroy cancer cells. An immune effector may be, for example, an antibody specific for some markers present on the surface of a tumor cell. Antibodies can function as effectors of therapy on their own or can recruit other cells that actually affect cell death. Antibodies can also be conjugated to drugs or toxins (chemotherapeutics, radionuclides, ricin A chain, cholera toxin, pertussis toxin, etc.) to function solely as targeting agents. Alternatively, the effector may be a lymphocyte that delivers surface molecules that interact directly or indirectly with the cell target. Various effector cells include cytotoxic T cells and NK cells, as well as genetically engineered variants of these cell types that have been modified to express chimeric antigen receptors.

Other complementary immunotherapies, such as GM-CSF to increase the number of bone marrow-derived innate immune system cells, low-dose cyclophosphamide or PI3K inhibitors to eliminate T regulatory cells that suppress innate and adaptive immunity, and 5FU, PI3K inhibitors or histone deacetylase inhibitors to eliminate bone marrow-derived suppressor cells, may be added to the above regimens to further enhance their efficacy.

Immunotherapy may also include administration of interleukins, such as IL-2, or interferons, such as INFα.

Examples of immunotherapies that can be combined with p53, ADP, and/or MDA-7 gene therapy and CD122/CD132 agonists include immune adjuvants (e.g., Mycobacterium bovis, Plasmodium falciparum, dinitrochlorobenzene, and aromatic compounds), cytokine therapy, gene therapy, and monoclonal antibodies. Therefore, it is considered that one or more anticancer therapies may be used in combination with the p53, ADP, and/or MDA-7 gene therapies described in the present application.

Other immunotherapies that may be combined with p53, ADP and/or MDA-7 gene therapy and CD122/CD132 agonists include immune checkpoint inhibitors, co-stimulatory receptor agonists, innate immune cell stimulators or innate immune activators. In certain aspects, immune checkpoint inhibitors are inhibitors of CTLA-4, PD-1, PD-L1, PD-L2, LAG-3, BTLA, B7H3, B7H4, TIM3, KIR, or A2aR. In certain aspects, at least one immune checkpoint inhibitor is an anti-killer-cell immunoglobulin-like receptor (KIR) antibody.

In some aspects, at least one immune checkpoint inhibitor is a human programmed cell death 1 (PD-1) axis binding antagonist. In certain aspects, the PD-1 axis binding antagonist is selected from the group consisting of a PD-1 binding antagonist, a PDL1 binding antagonist and a PDL2 binding antagonist. In some aspects, the PD-1 axis binding antagonist is a PD-1 binding antagonist. In certain aspects, the PD-1 binding antagonist inhibits the binding of PD-1 to PDL1 and/or PDL2. In particular, the PD- 1 binding antagonist is a monoclonal antibody or antigen-binding fragment thereof.

A co-stimulatory receptor agonist may be an anti-OX40 antibody, anti-GITR antibody, anti-CD 137 antibody, anti-CD40 antibody, or an anti-CD27 antibody. Stimulators of innate immune cells include, but are not limited to, KIR monoclonal antibodies, inhibitors of cytotoxic-inhibitory receptors, and toll-like receptors. Activators of innate immune cells such as natural killer (NK) cells, macrophages and dendritic cells include IDO inhibitors, TGF inhibitors and IL-10 inhibitors. An exemplary activator of innate immunity is indoximod.

The immunotherapy may include the suppression of T regulatory cells, myeloid-derived suppressor cells (MDSCs) and cancer-associated fibroblasts (CAFs). In some embodiments, the immunotherapy is a tumor vaccine (e.g., whole tumor cell vaccines, dendritic cell vaccines, DNA and/or RNA expression vaccines, peptides, and recombinant tumor-associated antigen vaccines), or adoptive cellular therapies (ACT) (e.g., T cells, natural killer cells, TILs, and LAK cells). The T cells and/or natural killer cells may be engineered with chimeric antigen receptors (CARs) or T cell receptors (TCRs) to specific tumor antigens. A chimeric antigen receptor (or CAR) used in the present invention may refer to any engineered receptor specific for an antigen of interest that, when expressed in a T cell or natural killer cell, confers the specificity of the CAR onto the T cell or natural killer cell. Once T cells or natural killer cells expressing the chimeric antigen receptor are generated using standard molecular techniques, they can be introduced into a patient, as in techniques such as adoptive cell transfer.

The immunotherapy may be a cancer vaccine including one or more cancer antigens, in particular a protein or an immunogenic fragment thereof, DNA or RNA encoding the cancer antigen, in particular a protein or an immunogenic fragment thereof, cancer cell lysates, and/or protein preparations from tumor cells. A cancer antigen used in this specification is an antigenic substance present in cancer cells. In principle, any protein produced in a cancer cell that is upregulated in cancer cells compared to normal cells or has an abnormal structure due to mutation can act as a cancer antigen. In principle, cancer antigens may be products of mutated or overexpressed oncogenes and tumor suppressor genes, products of other mutated genes, overexpressed or aberrantly expressed cellular proteins, cancer antigens produced by oncogenic viruses, oncofetal antigens, altered cell surface glycolipids and glycoproteins, or cell type-specific differentiation antigens. Examples of cancer antigens include the abnormal or overexpressed products of ras and p53 genes. Other examples include tissue differentiation antigens, mutant protein antigens, oncogenic viral antigens, cancer-testis antigens and vascular or stromal-specific antigens. Tissue differentiation antigens are those that are specific to a certain type of tissue. Mutant protein antigens are likely to be much more specific to cancer cells because normal cells should not contain these proteins. Normal cells display the normal protein antigen on their MHC molecules, whereas cancer cells display the mutant version. Some viral proteins are associated with cancer formation, and some viral antigens are also cancer antigens.

The immunotherapy may be an antibody, such as part of a polyclonal antibody preparation, or may be a monoclonal antibody. The antibody may be a humanized antibody, a chimeric antibody, an antibody fragment, a bispecific antibody or a single chain antibody. An antibody as disclosed herein includes an antibody fragment, such as, but not limited to, Fab, Fab' and F(ab')2, Fd, single-chain Fvs (scFv), single-chain antibodies, disulfide-linked Fvs (sdfv) and fragments including either a VL or VH domain. In some aspects, the antibody or fragment thereof specifically binds epidermal growth factor receptor (EGFR1, Erb-Bl), HER2/neu (Erb-B2), CD20, vascular endothelial growth factor (VEGF), insulin-like growth factor receptor (IGF-1R), TRAIL-receptor, epithelial cell adhesion molecule, carcino-embryonic antigen, prostate-specific membrane antigen, Mucin-1, CD30, CD33, or CD40.

Examples of monoclonal antibodies that may be used in combination with the compositions provided herein include, without limitation, trastuzumab (anti-HER2/neu antibody); Pertuzumab (anti-HER2 mAb); cetuximab (chimeric monoclonal antibody to epidermal growth factor receptor EGFR); panitumumab (anti-EGFR antibody); nimotuzumab (anti-EGFR antibody); Zalutumumab (anti-EGFR mAb); Necitumumab (anti-EGFR mAb); MDX-210 (humanized anti-HER-2 bispecific antibody); MDX-210 (humanized anti-HER-2 bispecific antibody); MDX-447 (humanized anti-EGF receptor bispecific antibody); Rituximab (chimeric murine/human anti-CD20 mAb); Obinutuzumab (anti-CD20 mAb); Ofatumumab (anti-CD20 mAb); Tositumumab-Il3l (anti-CD20 mAb); Ibritumomab tiuxetan (anti-CD20 mAb); Bevacizumab (anti-VEGF mAb); Ramucirumab (anti-VEGFR2 mAb); Ranibizumab (anti-VEGF mAb); Aflibercept (extracellular domains of VEGFR1 and VEGFR2 fused to IgGl Fc); AMG386 (angiopoietin-l and -2 binding peptide fused to IgGl Fc); Dalotuzumab (anti-IGF-IR mAb); Gemtuzumab ozogamicin (anti-CD33 mAb); Alemtuzumab (anti-Campath-l/CD52 mAb); Brentuximab vedotin (anti-CD30 mAb); Catumaxomab (bispecific mAh that targets epithelial cell adhesion molecule and CD3); Naptumomab (anti-5T4 mAb); Girentuximab (anti-Carbonic anhydrase ix); or Farletuzumab (anti-folate receptor). Other examples include antibodies such as Panorex^{™} (17-1A) (murine monoclonal antibody); Panorex (@ (17-1 A) (chimeric murine monoclonal antibody); BEC2 (ami-idiotypic mAb, mimics the GD epitope) (with BCG); Oncolym (Lym-1 monoclonal antibody); SMART M195 Ab, humanized 13' 1 LYM-1 (Oncolym), Ovarex (B43.13, anti-idiotypic mouse mAb); 3622W94 mAh that binds to EGP40 (17-1 A) pancarcinoma antigen on adenocarcinomas; Zenapax (SMART Anti-Tac (IL-2 receptor); SMART M195 Ab, humanized Ab, humanized); NovoMAb-G2 (pancarcinoma specific Ab); TNT (chimeric mAh to histone antigens); TNT (chimeric mAh to histone antigens); Gliomab-H (Monoclonals- Humanized Abs); GNI-250 Mab; EMD-72000 (chimeric-EGF antagonist); LymphoCide (humanized IL.L.2 antibody); and MDX-260 bispecific, targets GD-2, ANA Ab, SMART IDIO Ab, SMART ABL 364 Ab or ImmuRAIT-CEA.

Still other examples of antibodies include Zanulimumab (anti-CD4 mAb), Keliximab (anti-CD4 mAb); Ipilimumab (MDX-101; anti-CTLA-4 mAb); Tremilimumab (anti-CTLA-4 mAb); (Daclizumab (anti-CD25/IL-2R mAb); Basiliximab (anti-CD25/IL-2R mAb); MDX-1106 (anti-PDl mAb); antibody to GITR; GC1008 (anti-TGF-b antibody); metelimumab/CAT-l92 (anti-TGF-b antibody); lerdelimumab/CAT-l52 (anti-TGF-b antibody); ID11 (anti-TGF-b antibody); Denosumab (anti-RANKL mAb); BMS-663513 (humanized anti-4- 1BB mAb); SGN-40 (humanized anti-CD40 mAb); CP870,893 (human anti-CD40 mAb); Infliximab (chimeric anti-TNF mAb; Adalimumab (human anti-TNF mAb); Certolizumab (humanized Fab anti-TNF); Golimumab (anti-TNF); Etanercept (extracellular domain of TNFR fused to IgGl Fc); Belatacept (extracellular domain of CTLA-4 fused to Fc); Abatacept (extracellular domain of CTLA-4 fused to Fc); Belimumab (anti-B Lymphocyte stimulator); Muromonab-CD3 (anti-CD3 mAb); Otelixizumab (anti-CD3 mAb); Teplizumab (anti-CD3 mAb); Tocilizumab (anti-IL6R mAb); REGN88 (anti-IL6R mAb); Ustekinumab (anti-IL- 12/23 mAb); Briakinumab (anti-IL- 12/23 mAb); Natalizumab (anti-a4 integrin); Vedolizumab (anti-a4 b7 integrin mAb); Tl h (anti-CD6 mAb); Epratuzumab (anti-CD22 mAb); Efalizumab (anti-CDlla mAb); and Atacicept (extracellular domain of transmembrane activator and calcium-modulating ligand interactor fused with Fc).

Milling methods include physical impact, crushing, wet and dry milling, disintegration, freezing and cryo-milling, cutting, and the like. Milling methods include physical impact, crushing, wet and dry milling, disintegration, freezing and cryo-milling, cutting, and the like. The solution may be water or a liquid, buffer solution, isotonic solution, saline solution containing water as its main component, etc., but is not limited to a specific type.

In addition, when the composition includes two or more tumor tissues, the two or more tumor tissues may have the same or different milling particle sizes.

In addition, the present invention provides a kit for preventing or treating cancer including the composition. The kit according to the present invention may include, in addition to the composition described above, other components, compositions, solutions, devices, etc. that are usually necessary for the prevention or treatment of cancer without limitation, and in particular, may include instructions for proper use and storage of the composition according to the present invention.

The content of the tumor tissue or cancer cells in the composition of the present invention may be appropriately adjusted according to the symptoms of disease, the degree of progression of the symptoms, the condition of a patient, etc., and for example, it may be 0.0001 to 99.9 wt%, or 0.001 to 50 wt% based on the total weight of the composition, but is not limited thereto. The content ratio is a value based on the dry amount after removing the solvent.

The pharmaceutical composition according to the present invention may further include a suitable carrier, excipient and diluent commonly used in the preparation of a pharmaceutical composition. The excipient may be, for example, one or more selected from the group consisting of diluents, binders, disintegrants, glidants, adsorbents, moisturizers, film-coating materials, and controlled-release additives.

The pharmaceutical composition according to the present invention may be formulated and used in the form of external preparations such as powders, granules, sustained-release granules, enteric-coated granules, liquids, eye drops, ellipses, emulsions, suspensions, alcohols, troches, aromatic waters, limonades, tablets, sustained-release tablets, enteric-coated tablets, sublingual tablets, hard capsules, soft capsules, sustained-release capsules, enteric capsules, pills, tinctures, soft extracts, dry extracts, fluid extracts, injections, capsules, irrigation solutions, warnings, lotions, pastes, sprays, inhalants, patches, sterile injection solutions, or aerosols, according to conventional methods, and the external preparations may be formulated and used in the form of external preparations such as creams, gels, patches, sprays, ointments, warnings, lotions, liniments, pastes, or cataplasmas.

Examples of a carrier, an excipient and a diluent which may be included in the pharmaceutical composition according to the present invention may include lactose, dextrose, sucrose, oligosaccharides, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and the like.

When formulating, a commonly used diluent or excipient such as a filler, a bulking agent, a binder, a wetting agent, a disintegrant, or a surfactant is used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ionexchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, sodium carboxymethylcellulose, and the like may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro (OSI, OSIX, A, B, C, D, H, L), suppository base IV types (AB, B, A, BC, BBG, E, BGF, C, D, 299), suppostal (N, Es), Wecoby (W, R, S, M, Fs), and tegester triglyceride matter (TG-95, MA, 57) may be used.

Solid preparations for oral administration include tablets, pills, powders, granules, capsules, and the like, and such solid preparations are formulated by mixing the extract with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. As the non-aqueous solvent and the suspension, propylene glycol, polyethylene glycol, vegetable oil such as olive oil, and an injectable ester such as ethyl oleate, and the like may be used.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including the types of diseases of patients, the severity of the disease, the activity of drugs, sensitivity to drugs, administration time, administration route, excretion rate, treatment period, and simultaneously used drugs, and factors well known in other medical fields.

The pharmaceutical composition according to the present disclosure may be administered as an individual therapeutic agent, may be administered in combination with other therapeutic agents, may be administered sequentially or simultaneously with conventional therapeutic agents, and may be administered once or multiple times. In consideration of all of the above factors, it is important to administer an amount capable of obtaining the maximum effect with a minimum amount without side effects, which can easily be determined by those skilled in the art to which the present disclosure pertains.

The pharmaceutical composition of the present disclosure may be administered to a subject by various routes. All modes of administration may be considered, for example, oral administration, subcutaneous injection, intraperitoneal administration, intravenous injection, intramuscular injection, paraspinal space (intrathecal) injection, sublingual administration, buccal administration, rectal insertion, vaginal insertion, ocular administration, otic administration, nasal administration, inhalation, spray through the mouth or nose, dermal administration, transdermal administration, etc.

The pharmaceutical composition of the present disclosure is determined according to the type of drug as an active ingredient along with several related factors such as the disease to be treated, route of administration, patient's age, sex, weight, and severity of the disease. Specifically, the effective dose of the composition according to the present invention may vary depending on the patient's age, gender, and weight, and is generally 0.001 to 150 mg, preferably 0.01 to 100 mg, per 1 kg of body weight daily, every other day, at intervals of 3 to 4 days, or divisionally into 1 to 3 times a day. However, the dosage may increase or decrease depending on the route of administration, the severity of disease, gender, weight, age, etc., and thus the above dosages do not limit the scope of the present invention in any way.

In the present invention, the term "subject" refers to a subject in need of treatment for a disease, and more specifically, may be a mammal of humans or non-human primates, mice, rats, dogs, cats, horses, cattle, etc., but is not limited thereto.

In the present invention, the term "administration" refers to the provision of a predetermined composition of the present invention to a subject by any suitable method.

In the present invention, the term "prevention" refers to all actions that inhibit or delay the onset of a target disease, the term "treatment" refers to all actions that improve or beneficially change a target disease and metabolic abnormalities thereof by the administration of the pharmaceutical composition according to the present invention, and the term "amelioration" means any action that reduces a parameter related to a target disease, such as the degree of symptoms, by administering a composition according to the present invention.

In addition, the present invention provides a method of preparing the composition according to the present invention, the method including: a step (S1) of isolating tumor tissue or cancer cells from a cancer patient; and

a step (S2) of applying one or more treatments selected from the group consisting of (a) and (b) below to the isolated tumor tissue or cancer cells,
(a) radiation irradiation; and
(b) a cancer immunotherapy drug.

The tumor tissue or cancer cells may be obtained through a procedure to remove them, or the tumor tissue or cancer cells removed during the treatment or diagnosis process may be utilized. For example, by using tissue specimens for tissue examination to determine whether there is a malignant tumor in the body, rather than discarding the specimens, after microscopic examination or other tissue examination confirmation, the patient's pain associated with the removal of cancerous tissues may be reduced.

In addition, if the amount of tissue removed or reused after tissue examination is small, the tissue may be cultured to increase the amount needed. In cases where culture is not easy, a small amount of excised tissue may be milled, diluted in water, and increased in volume for ex vivo treatment.

Therefore, the method may further include a step of milling the tumor tissue and diluting or dissolving it in a solution after the step (S1).

The extracted tissue may be separated into the amount required for extracorporeal abscopal induction treatment, labeled, and then treated.

Hereinafter, preferred examples are presented to help understand the present invention. However, the following examples are provided only to help understand the present invention more easily, and the contents of the present invention are not limited by the following examples.

### [Example]

### Example 1. Preparation of composition having abscopal effect using radiation irradiation

As the first means to achieve the abscopal effect for cancer or tumor treatment, the tumor tissue was separated from a subject, and then the extracted tissue was treated with radiation irradiation and then re-administered. Specifically, when irradiating an extracted tissue specimen, a single dose of radiation can be irradiated. The irradiation dose at this time can be within the range of 1Gy to 500Gy, and is administered once or in multiple doses. Alternatively, instead of irradiating a single dose of radiation, multiple-dose equivalents can be irradiated and mixed, and two or more total doses can be irradiated and mixed. In this case, excised tissue specimens are classified and irradiated, and then the tissues that have undergone radiation treatment are mixed to produce a composition for administration.

For example, two (A, B) radiation irradiation dose mixtures can be prepared under the following conditions:
- Tissue A: 1 to 50 Gy/total irradiation dose
- Tissue B: 50 to 500 Gy/total irradiation dose

Three (A, B, C) radiation irradiation dose mixtures can be manufactured under the following conditions:
- Tissue A: 1~30 Gy/total irradiation dose
- Tissue B: 30 to 100 Gy/total irradiation dose
- Tissue C: 100 to 1000 Gy/total irradiation dose

The above examples present 2 to 3 total dose mixtures, and a larger number of total dose mixture compositions can be made if necessary.

### Example 2. Preparation of composition having abscopal effect using immunological treatment

In this example, a composition capable of exerting an abscopal effect is produced by isolating tumor tissue from a subject and then performing immunological treatment on the extracted tissue. When immunologically treating a tissue specimen extracted from a subject, the tissue specimen may be treated as a single agent, or separated into two or more tissue specimens and treated with two or more immunizing agents.

When immunologically treating the separated tissue specimens, the separated tissue specimens are made such that the tissue cells are killed or weakened and thus cannot grow or proliferate, and preferably such that immune cells can easily recognize the tissue cells of the immunologically treated composition.

Immunological treatment encompasses the use of immune effector cells and molecules to target and destroy the extracted cancer and tumor tissue cells.

The immune effector may be, for example, an antibody specific for some marker on the surface of a tumor cell. Antibodies may be used alone as effectors of therapy, or may recruit other cells to achieve actual cell death. Antibodies may also be conjugated to drugs or toxins (radionuclides, ricin A chain, cholera toxin, pertussis toxin, etc.) and used primarily as targeting agents. As another example, the effector may be a lymphocyte-bearing surface molecules that interact directly or indirectly with target tumor cells. Various effector cells include cytotoxic T cells, NK cells, and the like. It also encompasses the use of apoptotic cells in immunological treatment.

Immunological preparations may be administered by mixing one or more of previously developed preparations.

As an example, when two (A, B) extracted specimens are treated with an immunological therapeutic agent, a composition is created by inducing apoptosis or attenuation of cancerous tissues in the extracted tissue specimen A through the immunological therapeutic agent treatment. Treatment of the excised tissue specimen B is performed by performing an immunological treatment other than the immunological treatment for specimen A, thereby inducing the apoptosis or attenuation of cancer cells in the specimen B tissue, resulting in the production of a composition. The compositions of the treated specimens A and B are administered to the subject separately or together as a mixture.

When there are three or more immunological methods to be treated, the treatment is performed in the same division method as the two immunological treatment methods, and the prepared compositions are administered to the subject individually or together as a mixture.

In another embodiment of the present invention, A composition of each of extracted cancerous tissues treated by at least one method selected from the group consisting of radiation irradiation treatment, immunological treatment and the like, or a composition made by integrating the compositions can be administered into the subject's body. If necessary, each of the compositions prepared by treating the tissues in one or more ways, or a composition prepared by mixing two or more of the compositions is administered to the patient.

The timing of administration is not separately specified. When administered with an immunizing agent, it may be administered 30 minutes to 2 hours before the administration of the immunizing agent, simultaneously with the immunotherapy agent, or 30 minutes to 1 week after the administration of the immunotherapy agent.

In the present invention, "cancer" includes all types of solid tumors and all types of blood cancer.

To apply the present invention to blood cancer, the extracted blood was separated by centrifugation, mesh, filter, etc., and only cancerous tissues were separated and processed in the same way as in solid cancer tissues, thereby producing an abscopal vaccine.

### Example 3. Confirmation of anticancer effect of composition (IVAM) having abscopal effect manufactured using radiation treatment

The In vitro Abscopal Method (IVAM) of the present invention is expected to activate the subject's anti-tumor immunity and induce the abscopal effect by administering radiation-irradiated tumor cells. Accordingly, the present inventors confirmed the effectiveness of the present invention using breast cancer animal models with low immunogenicity and an immune-suppressive tumor microenvironment.

### 3-1. Experimental method

Specifically, the in vitro experiment was conducted as follows: 4T1 murine triple-negative breast cancer cells were seeded at a density of 2 × 10⁵ cells in a 60 mm culture dish and 1 × 10⁴ cells in a 24-well plate, and then radiation irradiation (50 Gy) was performed the next day. Cell morphology was observed under a microscope on days 2, 4, and 7 after the irradiation, and cell viability was measured by performing MTT.

*In vivo* experiments were conducted in compliance with animal ethics rules according to the Seoul National University Bundang Hospital IACUC Animal Experiment Plan (BA-2112-333-009-01). First, to create tumor animal models, 4T1 murine triple-negative breast cancer cells (6×10⁵ cells at right hindlimb or 1×10⁵ cells at left flank) were injected into the hindlimb and flank of 6-week-old immune-competent BALB/c mice. In the present invention, the 4T1 cell can be injected with 4T1-Luc cells expressing luciferase (same as the following example), and when luciferin is injected into the mice, the expressed luciferase (enzyme) uses it to emit light. The light can be detected by in vivo bioluminescence imaging to monitor the size and location of the tumor in the mice. Seven days after injection, the tumor was confirmed to have grown steadily, and experimental groups were assigned. The experimental groups were divided into a total of 4 groups, and 5 mice were assigned to each group: control group; IVAM administration group; radiation treatment group; and IVAM+radiation-combined treatment group. Specifically, IVAM administration was performed by culturing 4T1 cells irradiated with 50 Gy for a week, and then obtaining a cell supernatant (with dead cells) and administering 200 µl of the cell supernatant intravenously once (on Day 10). In addition, 8 Gy was administered three times a week (Day 10, 12 and 14) using a 6 MeV electron beam for radiation treatment, for a total of 24 Gy.

The tumor size and body weight of each experimental group were measured and recorded every Monday, Wednesday, and Friday, and the tumor growth inhibition effect, and the abscopal effect by radiation were compared and analyzed using IVIS imaging on the 4th and 28th days after tumor implantation.

### 3-2. High-dose radiation sensitization effect in 4T1 murine triple-negative breast cancer cell line

To measure the cell viability of 4T1 cells when exposed to high-dose radiation (50 Gy), a cytotoxicity test (MTT assay) was performed, and changes in cell shape were observed under a microscope.

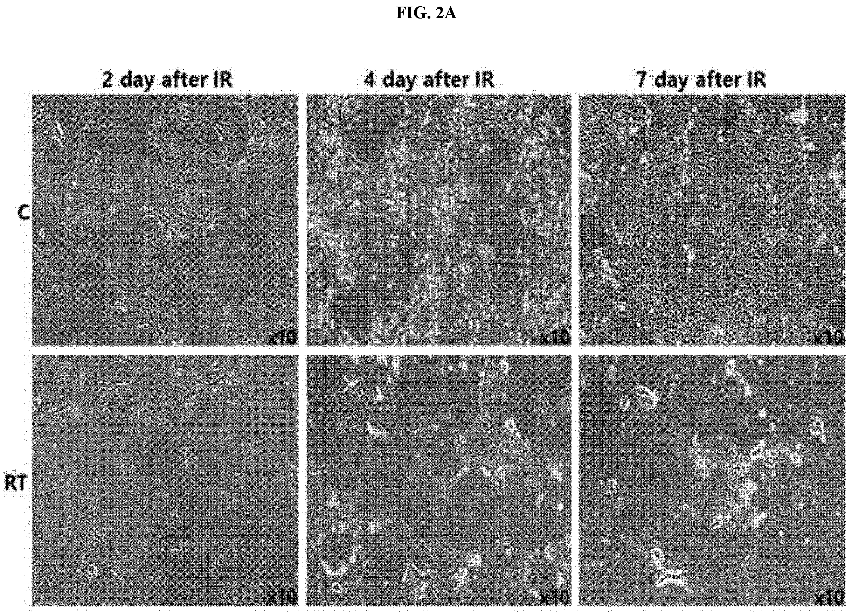

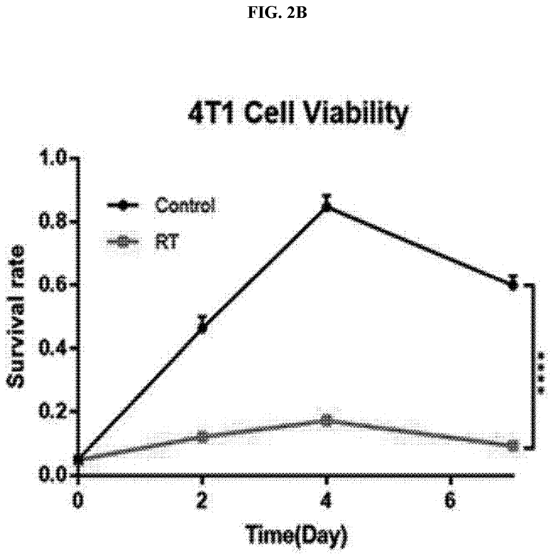

As a result, as shown in FIGS. 2A and 2B, the cancer cells irradiated with radiation were observed to have damaged cell membranes, compared to the control group, and the fraction of surviving cells was also significantly lower than the control group, confirming the sensitization effect by radiation (p<0.0001).

### 3-3. Synergistic anticancer effect and abscopal effect confirmed by combined use of IVAM and radiation

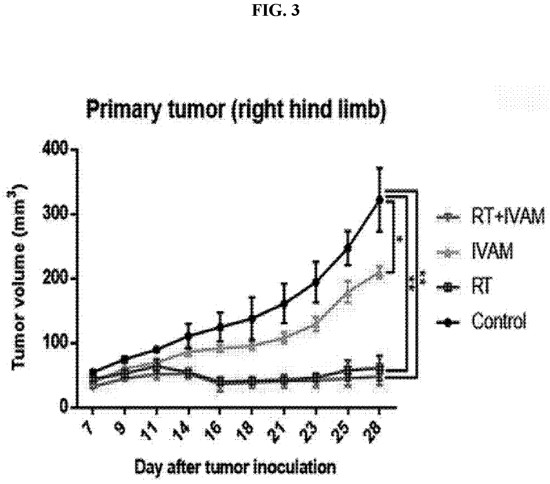

As a result of observing tumor growth for 28 days after tumor inoculation, it was confirmed that tumor growth was significantly (p<0.05) delayed in an IVAM single-administered group compared to a control group. In addition, when treated with a combination of radiation and IVAM, the synergistic effect where tumor growth was delayed more significantly (p<0.01) compared to the control group (FIG. 3) was confirmed (FIG. 3).

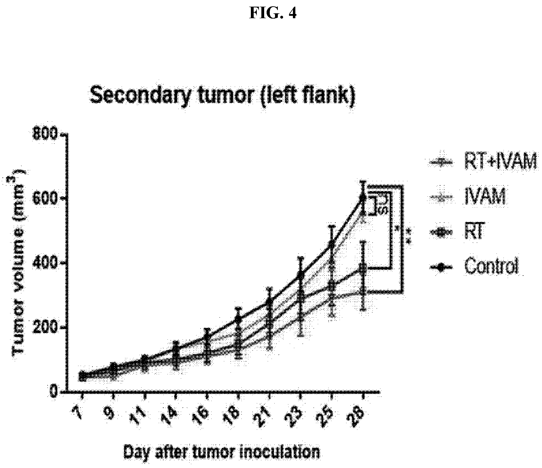

In addition, the growth of the tumor (secondary tumor) on the left flank that was not irradiated was significantly (p>0.05, p<0.01) suppressed in a radiation monotreatment group or a radiation + IVAM combination treatment group, respectively, compared to the control group. The IVAM single-administered group showed a slight but statistically insignificant decrease in the tumor on the left flank (FIG. 4).

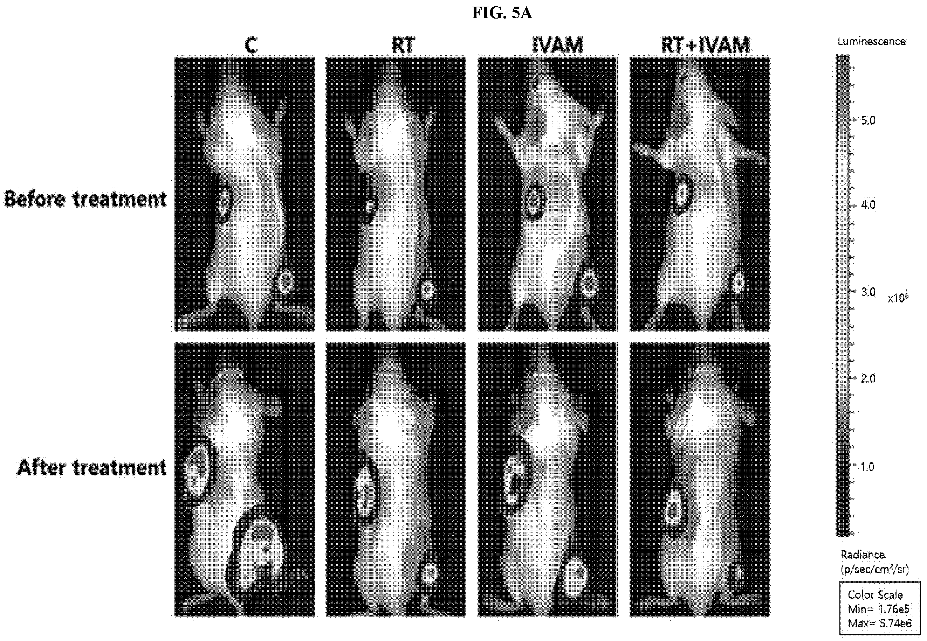

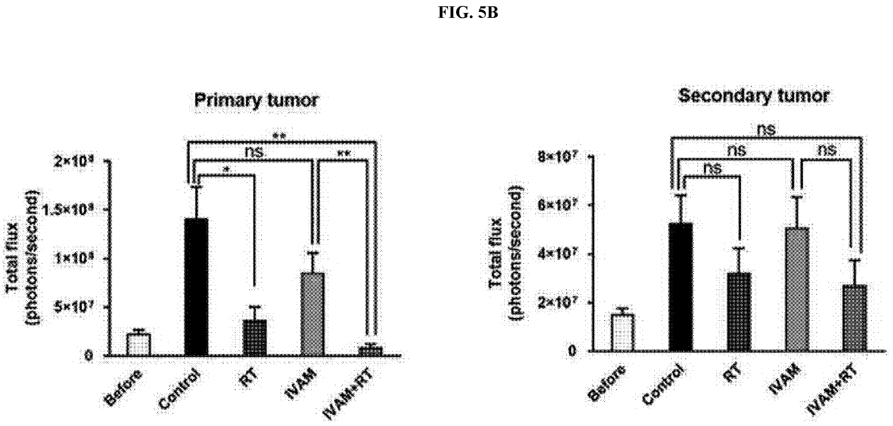

In addition, IVIS imaging using the Luciferase system was used to compare and analyze the results on the 4th and 28th days after tumor implantation. As a result of measuring the size of the tumor directly, it was confirmed that a synergistic anticancer effect was exerted when IVAM and radiation were used in combination, similar to the previously confirmed results. In addition, it was confirmed that a synergistic tumor suppression effect was exerted also in secondary tumors, which were not radiation-irradiated, according to the combined use of IVAM and radiation, confirming the exertion of the abscopal effect (FIGS. 5A and 5B).

The results show that when the IVAM of the present invention is used in combination with radiation, the anticancer effect is significantly increased compared to each monotherapy, and an even better anticancer effect can be achieved through the abscopal effect.

### 3-4. Confirmation of toxicity of combined IVAM and radiation

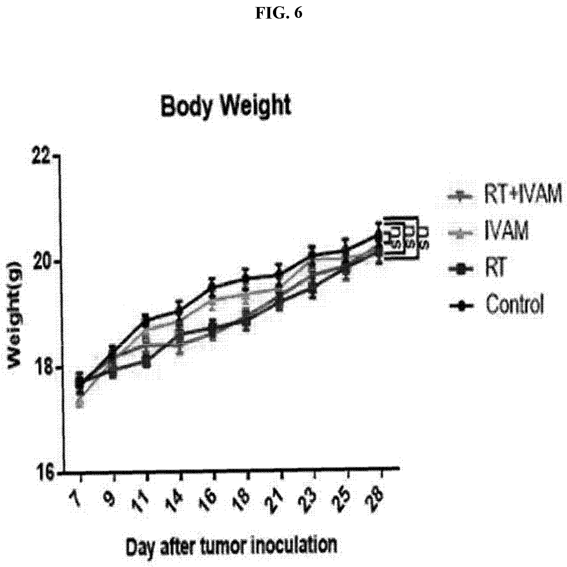

When the body weight of mice was measured for 28 days, no significant weight loss was observed due to IVAM and radiation compared to the control group (FIG. 6). The results suggest that the combined use of IVAM and radiation of the present invention can only exert excellent anticancer effect and abscopal effect without causing side effects such as toxicity to the subject.

### Example 4. Confirmation of combined effects of IVAM, radiation therapy, and cancer immunotherapy drug

In this example, the combined effect of IVAM and other anticancer therapy according to the present invention was confirmed. Specifically, the anticancer effect was compared by combining IVAM with radiation therapy and/or a cancer immunotherapy drug (an immune checkpoint inhibitor).

### 4-1. Experimental method

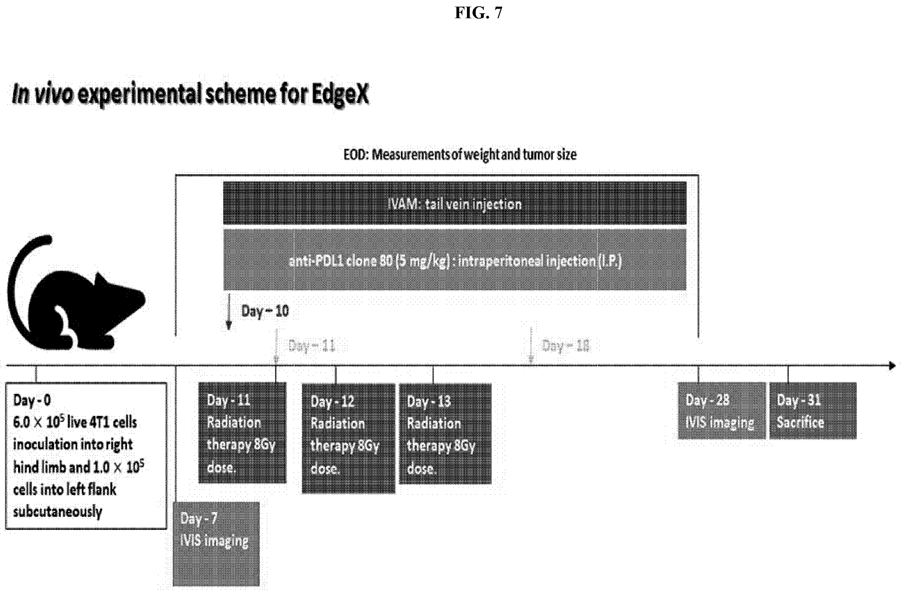

The animal experiment schedule according to this example is shown in FIG. 7. All animal experiments were conducted in compliance with animal ethics regulations according to the Seoul National University Bundang Hospital IACUC Animal Experiment Plan (BA-2112-333-009-01).

Specifically, the experiment was conducted as follows: 4T1 murine triple-negative breast cancer cells (6×10⁵ cells in the right hind leg or 1×10⁵ cells in the left flank) were injected into the hind limb and flank of 6-week-old immune-competent BALB/c mice. Seven days after injection, the tumor was confirmed to have grown steadily, and the experimental group was divided into eight groups, with five mice assigned to each group: ① Control group, ② Radiation treatment (RT) group, ③ IVAM-administered group, ④ IVAM-administered group + radiation combination treatment group, ⑤ a-PD-L1-administered group, ⑥ a-PD-L1 + radiation combination treatment group, ⑦ IVAM-administered group + a-PD-L1-administered group, and ⑧ IVAM-administered group + a-PD-L1-administered group + radiation triple-combination treatment group. Here, each anticancer therapy was performed as follows:
1) IVAM: ① 4T1 cells irradiated with 5, 25, 50, and 100 Gy were cultured for a week, and then the cell supernatant (with 1x10⁵ dead cells/injection) was collected at a ratio of 1:1:1:1, and 200 µl of the cell supernatant was intravenously injected once (on Day 10).
2) Radiation treatment (RT): Using X-RAD320 equipment from Precision X-ray Co., 8 Gy was administered three times a week (Day 11, 12 and 13), and finally a total of 24 Gy was administered. Radiation was administered only to the right hind limb of the mice.
3) a-PD-L1 (anti-Programmed Death-Ligand 1 antibody): 5 mg/kg concentration, 100 µl each, administered intraperitoneally twice (Day 11, 18).

Tumor size and body weight of mice in each group were measured and recorded every Monday, Wednesday, and Friday. On the 7th and 28th days after tumor implantation, IVIS imaging was used to compare and analyze the tumor growth inhibition effect by each treatment and the abscopal effect by radiation. On the 19th day after tumor implantation, orbital blood was collected from the mice, and the serum separated through centrifugation was used to measure interferon-γ (IFN-γ) and interferon-β (IFN-β) levels through multiplex immunoassay. All mice were euthanized on day 31 after tumor implantation. At this time, primary (tumor of the right hind limb irradiated with radiation) and secondary (tumor of the left flank not irradiated with radiation) tumor tissue and spleen, draining lymph node (dLN), and lung tissue were extracted from the mouse hind limb and flank, respectively. A portion of each tumor tissue and lung tissue was fixed with 4% paraformaldehyde immediately after extraction, and the other tumor and spleen tissues were used for flow cytometry analysis (FACS) after single cell isolation after extraction. The number of metastatic lung nodules observed in the fixed lung tissue was counted using a dissecting microscope. Statistical analysis was performed using one-way ANOVA (Tukey's multiple comparison tests) and unpaired two-tailed Student's t-tests of PRISM statistical analysis and graphing software (GraphPad 8).

### 4-2. Observation of tumor growth inhibition effect of IVAM, a-PD-L1, and radiation combination therapy

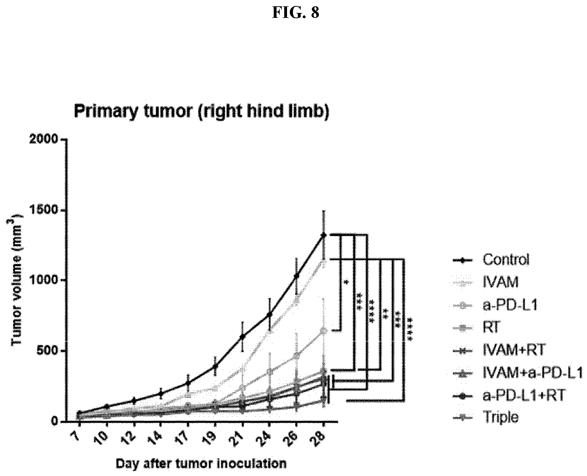

The results of tumor growth observation in the right hind limb (primary tumor) irradiated with radiation are shown in FIG. 8. Tumor growth was observed for 28 days after tumor inoculation into mice. First, in the right hind limb (primary tumor) that was irradiated with radiation, it was confirmed that tumor growth in the triple combination (IVAM + a-PD-L1 + radiation) group was significantly (p<0.0001) delayed compared to the control group. In addition, it was confirmed that, in all of the RT monotreatment group (p<0.001), the IVAM + RT group, the IVAM + a-PD-L1 group, and the a-PD-L1 + RT group (all three groups p<0.0001), the tumor size was somewhat larger than in the triple combination group, but tumor growth was significantly suppressed compared to the control group. Compared with the control group, a slight tumor growth delay was observed in the IVAM monotreatment group, but there was no significant difference.

Compared with the IVAM monotreatment group, it was confirmed that tumor growth was significantly delayed in the RT monotreatment group (p≤<0.01), the IVAM + RT group, the IVAM + a-PD-L1 group, the a-PD-L1 + RT group (all three groups p<0.001), and the triple combination group (p<0.0001).

When comparing the a-PD-L1 monotreatment group with the IVAM + a-PD-L1 group, there was no significant difference (p=0.6380), but the tumor size tended to be smaller when combined treatment was performed. However, there was no difference in tumor size between the RT monotreatment group and the IVAM + RT group.

As a result, the results of comparing the monotreatment, dual-combination, and triple-combination effects of IVAM, a-PD-L1, and RT confirmed that the anticancer treatment effect was strongest in the triple-combination group that combined all three treatments.

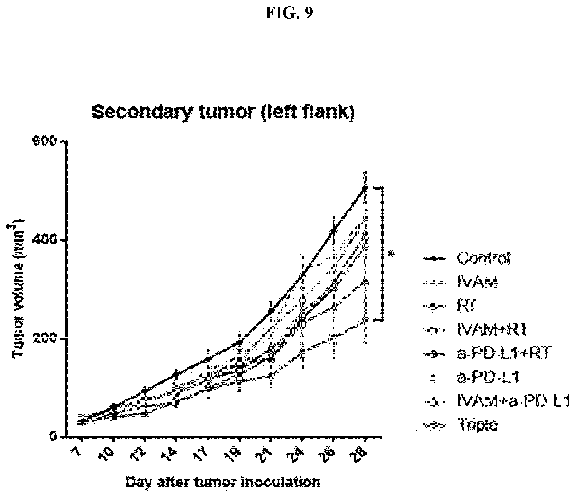

The results of observing the growth of secondary tumors are shown in FIG. 9. As shown in the drawing, the growth of the tumor (secondary tumor) in the left flank that was not irradiated with radiation was compared with the control group, and the tumor growth was significantly delayed in the triple combination group of IVAM + a-PD-L1 + RT, confirming the abscopal effect (p<0.05). There was no significant difference in tumor size between the different treatment groups.

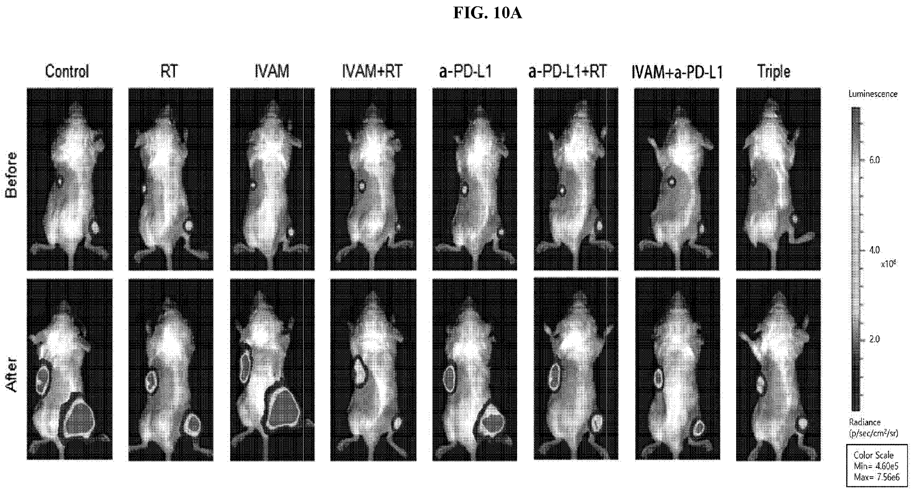

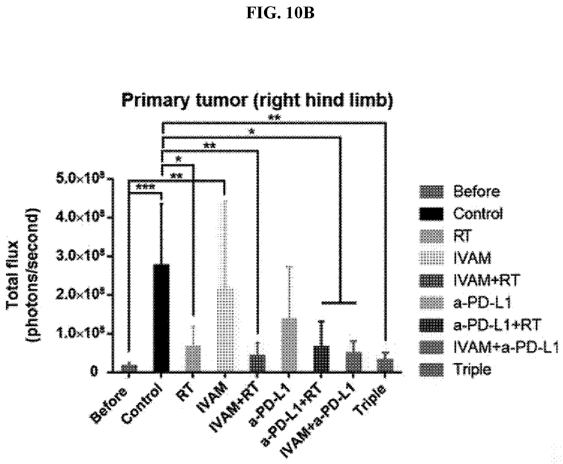

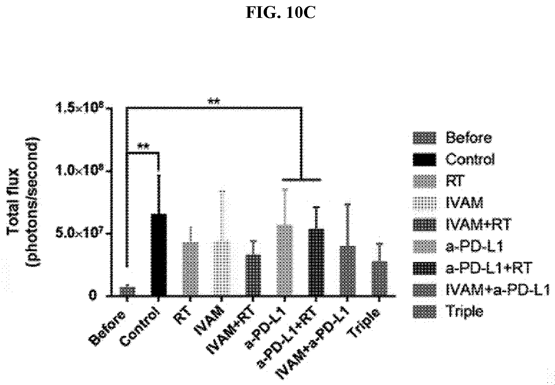

**In** addition, tumors were compared and analyzed on 4 days and 28 days after tumor implantation using IVIS imaging using the Luciferase system. As a result, a tumor growth inhibition effect pattern similar to the previously confirmed tumor growth inhibition effect comparison results (FIGS. 8 and 9) was confirmed (FIGS. 10A to 10C). That is, tumor growth was suppressed in all groups compared to the control group, and in particular, the anticancer effect of the triple combination of IVAM + a-PD-L1 + RT was confirmed to be the strongest.

In summary of the above results, in the case of tumor growth of the primary tumor, a decrease in tumor size was observed in the IVAM monotreatment group compared to the control group, and a tendency for tumor size to decrease further in the IVAM + a-PD-L1 combination therapy group compared to the a-PD-L1 monotherapy group was observed. In particular, it was confirmed that tumor growth was most strongly inhibited in the triple combination group of IVAM + a-PD-L1 + radiation. In addition, in the case of secondary tumors that did not directly receive radiation, an abscopal effect was observed in the triple combination group. These results demonstrate that the combination therapy of IVAM and radiation therapy and a cancer immunotherapy drug according to the present invention not only achieves synergistic anticancer effects but also exerts a more potent anticancer effect through the abscopal effect. In addition, as a result of measuring the changes in the immune cell population of the animal models, the primary tumor growth inhibition effect by the triple combination was confirmed to be due to the increase in CD8+ T cells and CD8+ effector memory T cells in the tumor microenvironment and the increase in antitumor immune effect due to the decrease in Tregs. In particular, the increase of Ki67+ CD8+ T cells and Ki67+ CD4+ T cells by the triple combination was confirmed also in the spleen. Furthermore, in mice treated with triple combination therapy, an increase in IFN-γ, which contributes to the antitumor immune effect, was observed, which is an important clue to the abscopal effect of triple combination therapy.

### 4-3. Toxicities of IVAM, a-PD-L1, and radiation combination therapy

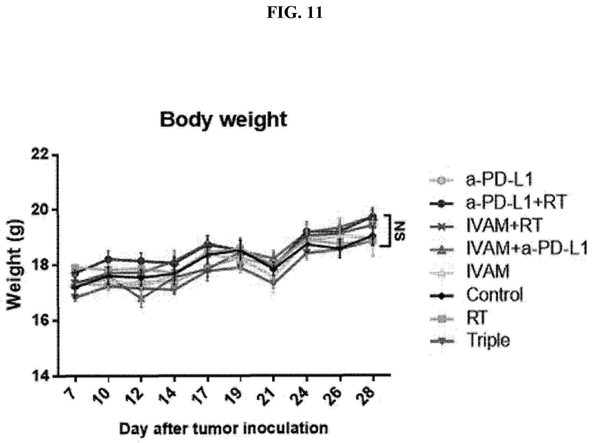

To determine whether there were any side effects from the combination of IVAM therapy, a-PD-L1, and radiation according to the present invention, the weight changes of mice were checked over time during the treatment course. As a result of measuring the body weight of mice for 31 days, it was confirmed that there was no significant weight loss in any group treated with IVAM, a-PD-L1, and/or radiation compared to the control group (FIG. 11). The result suggests that the combined use of the IVAM, cancer immunotherapy drug and radiation of the present invention can exert excellent anticancer and abscopal effects without causing side effects such as toxicity to the subject.

### 4-4. Confirmation of lung metastasis inhibition effect of IVAM, a-PD-L1, and radiation combination therapy

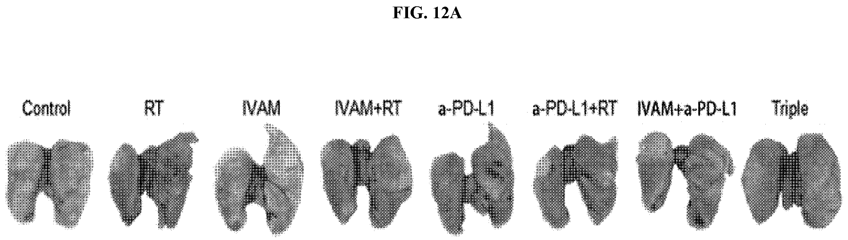

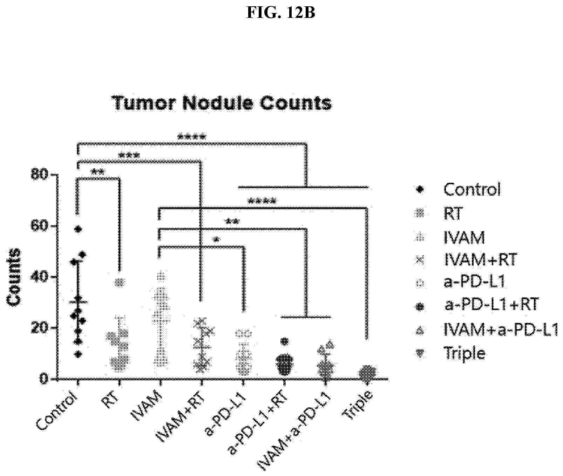

To confirm the metastasis inhibition effect of the triple combination of the IVAM, cancer immunotherapy drug, and radiation according to the present invention, mice were inoculated with tumors, lung tissues were extracted from mice euthanized on day 31, and metastatic lung nodules observed on the tissue surface were measured. As a result, in the control group, a very high number of nodules were observed, confirming that lung metastasis had occurred. On the other hand, a significant decrease in metastasis was confirmed in the a-PD-L1 monotreatment group, the a-PD-L1 + RT dual combination group, the IVAM + a-PD-L1 dual combination group, and the triple combination group (all p<0.0001). Metastasis was significantly reduced in the IVAM + RT dual combination group (p<0.001) and the RT monotreatment group (p<0.01), but there was no significant difference from the IVAM monotreatment group (FIGS. 12A and 12B).

The greatest difference was observed in the triple combination group (p<0.0001) compared to the IVAM monotreatment group, and differences were also found in the a-PD-L1 + RT dual combination group and the IVAM + a-PD-L1 dual combination group (both p<0.01), and the a-PD-L1 monotreatment group (p<0.05). From this, it was confirmed that the lung metastasis inhibition effect significantly increases even when only two or more of the three therapies, IVAM, RT, and a-PD-L1, of the present invention are combined, and in particular, a synergistic metastasis inhibition effect is shown when all of the therapies are combined.

### 4-5. Confirmation of changes in distribution of immune cells in primary tumors according to IVAM, a-PD-L1, and radiation combination treatment

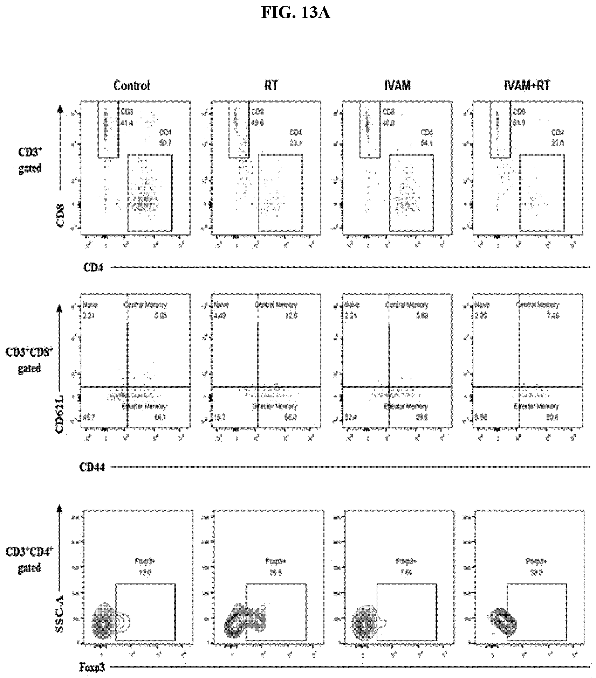

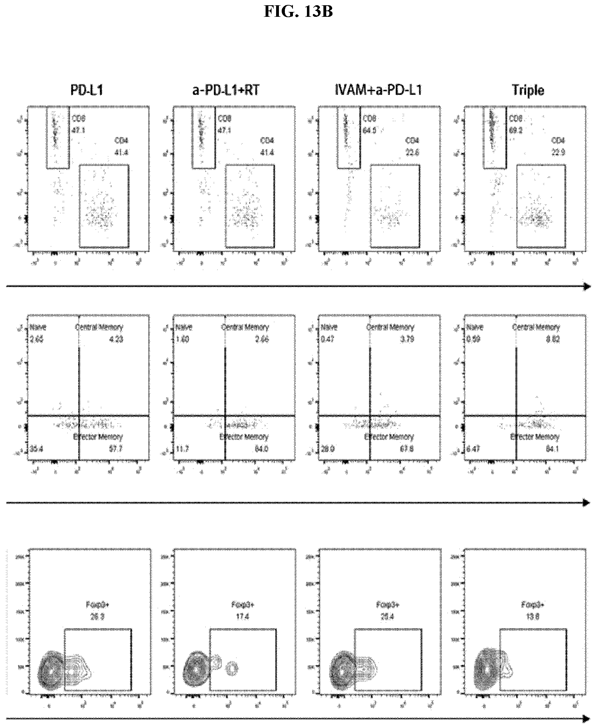

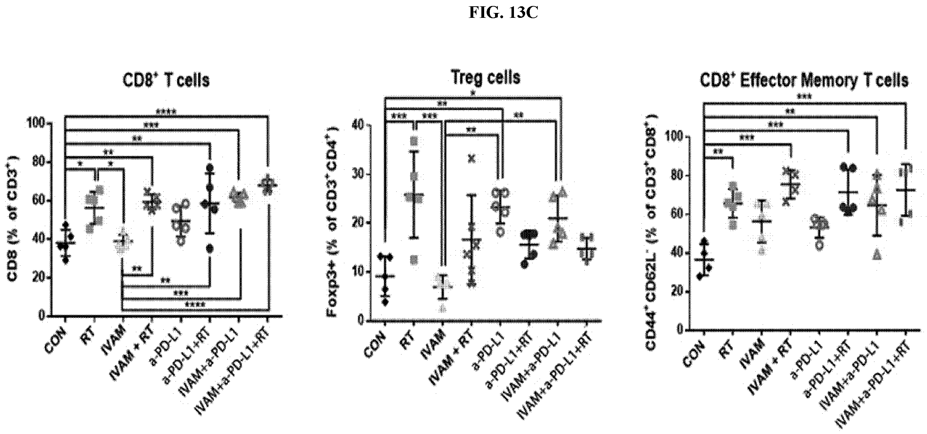

The results of flow cytometry analysis of population changes in three types of immune cells (CD8+ T cell, Treg and CD8+ effector memory T cell) in the tumor microenvironment of primary tumors (hind limb tumors) are shown in FIGS. 13A to 13C.

First, it was confirmed that the number of CD8⁺ T cells attacking tumor cells significantly increased in the IVAM + a-PD-L1 + radiation triple combination group compared to the control group (p<0.0001), and CD8⁺ T cells significantly increased in the RT monotreatment group (p<0.05), the IVAM + RT dual combination group (p<0.01), the a-PD-L1 + RT dual combination group (p<0.01), and IVAM + a-PD-L1 dual combination group (p<0.001).

There are previous research results showing that immunosuppressive regulatory T cells (T_{regs}) that suppress the cytotoxic activity of CD8⁺ T cells increase due to radiation. Consistent with this, the number of T_{regs} increased in the RT monotreatment group compared to the control group, and the number of T_{regs} increased in the IVAM + RT group compared to the IVAM monotreatment group. On the other hand, a decrease in T_{regs} was observed in the triple combination group, compared to the monotreatment group (IVAM +a-PD-L1 group) that did not receive RT.

In addition, in the case of CD8+ effector memory T cells that enable memory T cells with memory for a specific antigen to attack tumor cells with the same antigen, a significant increase was observed in all of the RT monotreatment group, the IVAM + a-PD-L1 dual combination group (p<0.01), the IVAM + RT dual combination group, the a-PD-L1 + RT dual combination group, and the triple combination group (p<0.001) compared to the control group.

The results demonstrate that the triple combination of IVAM + a cancer immunotherapy drug + radiation increases the number of CD8+ T cells with anticancer activity and decreases the number of T_{regs} that suppress the activity of CD8+ T cells, thereby enhancing the anticancer immune effect.

In summary, it can be seen that in the triple combination group, the number of CD8+ T cells and CD8+ effector memory T cells increases and the number of T_{regs} decreases, thereby enhancing the antitumor immune effect.

### 4-6. Confirmation of changes in distribution of immune cells in spleen according to IVAM, a-PD-L1, and radiation combination treatment

The spleen contains a variety of immune cells, including lymphocytes such as T cells, as well as monocytes that later differentiate into macrophages and dendritic cells, and thus play an important role in regulating the immune system throughout the body. Accordingly, the effect of combined use of the IVAM, cancer immunotherapy drug, and/or radiation according to the present invention on the distribution of immune cells in the spleen was confirmed.

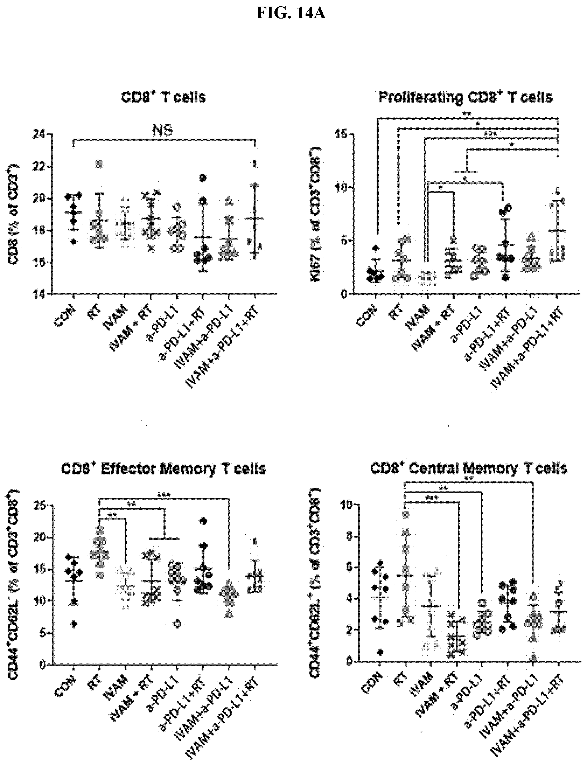

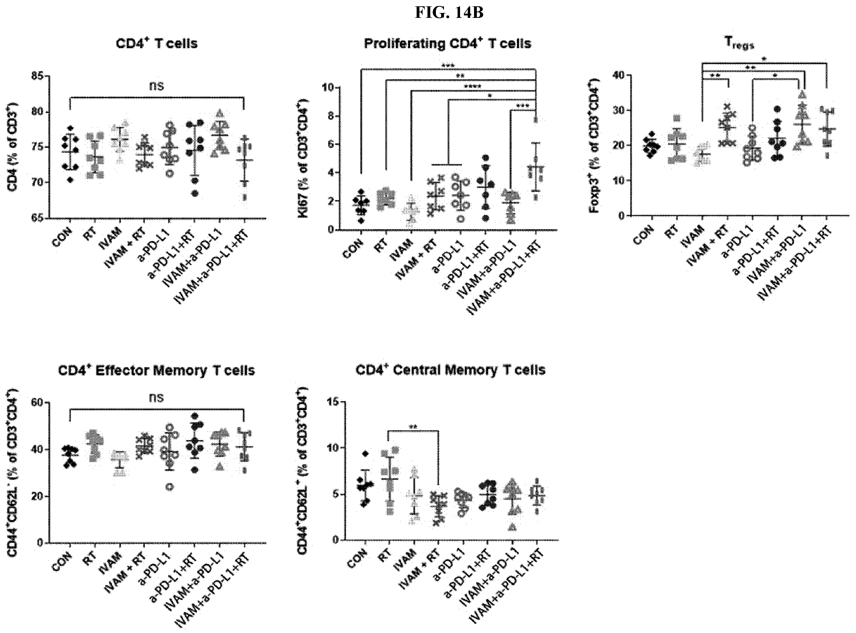

The results are shown in FIGS. 14A and 14B. First, when observing the population changes of CD8+ and CD4+ T cells, no significant differences were observed in any group. However, the clusters of Ki67+ CD8+ T cells and Ki67+ CD4+ T cells expressing Ki67, a cell proliferation marker, were found to significantly increase in the triple combination group compared to the control group. The result suggests that the abscopal effect of the triple combination of the IVAM, cancer immunotherapy drug, and radiation according to the present invention is related to changes in the distribution of immune cells in the spleen.

In the case of T_{regs}, the number of T_{regs} increased in the RT monotreatment group compared to the control group; the IVAM monotreatment group compared to the IVAM + RT combination group; and the a-PD-L1 + RT group compared to the a-PD-L1 monotreatment group, as in the patterns shown in FIG. 13A to 13A. However, in the triple combination group, a slight decrease in T_{regs} was observed, compared to the IVAM + a-PD-L1 dual combination group.

### 4-7. Confirmation of changes in cytokines in serum according to IVAM, a-PD-L1, and radiation combination treatment

The effect of combined use of the IVAM, cancer immunotherapy drug, and/or radiation according to the present invention on the level of cytokines in serum was confirmed. For this, serum was collected by collecting orbital blood from mice on day 19 after tumor inoculation, and the levels of two interferons, IFN-β and IFN-γ, in the serum were measured.

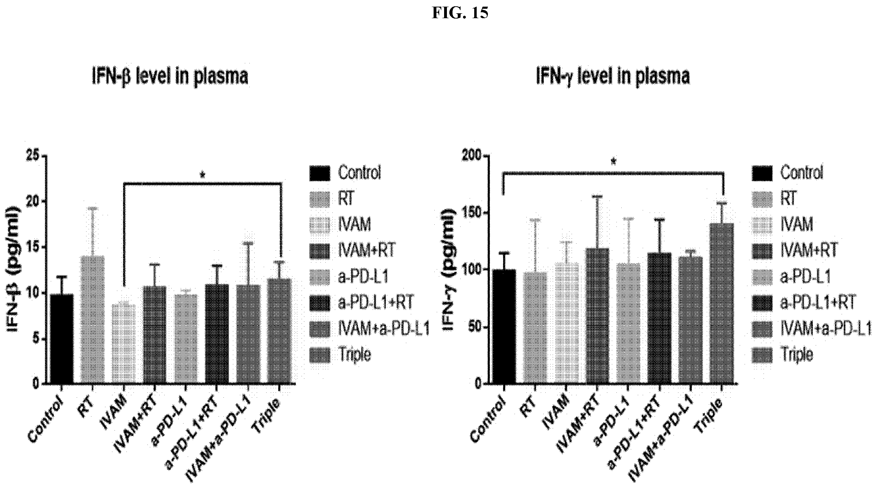

As a result, a significant increase in IFN-γ was observed in the triple combination group (IVAM + a-PD-L1 + radiation) compared to the control group (p<0.05), and an increasing trend of IFN-β was observed in all groups that received RT, compared to the control group, as shown in FIG. 15.

The results suggest that the radiation therapy increases the level of interferon, which is consistent with the immune enhancement and interferon-increasing effects of RT known from previous studies. In particular, in the case of IFN-γ, it was found that the increase was greater in the triple combination group applied with a combination of IVAM and a-PD-L1 together with RT treatment. Furthermore, the overall increase in IFN-γ in the body according to the triple therapy is considered to be the basis for explaining the growth delay effect and abscopal effect of primary tumors by the triple therapy.

### Example 5. Confirmation of long-term survival trial results by combination treatment of IVAM, radiation therapy, and cancer immunotherapy drug

In this example, the effects of the combined use of IVAM and other anticancer therapy according to the present invention were confirmed over a long period. Specifically, IVAM was combined with radiation therapy and/or the cancer immunotherapy drug (immune checkpoint inhibitor) to compare the anticancer effect.

### 5-1. Experimental method

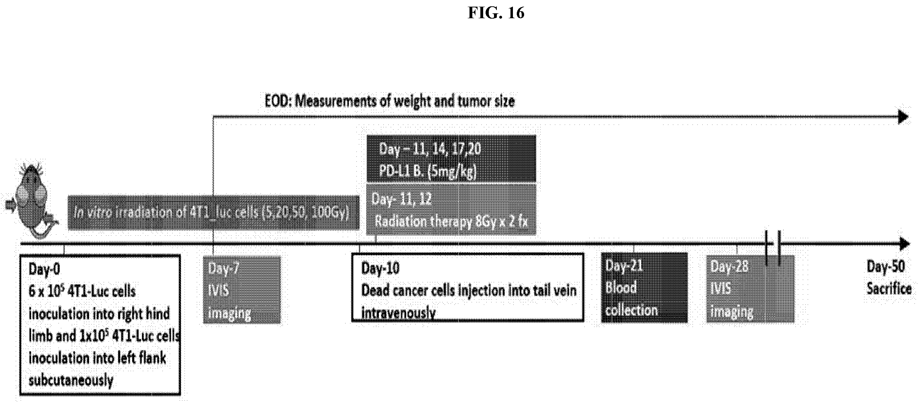

The animal testing schedule for the long-term survival rate and toxicity confirmation experiments according to this example is shown in FIG. 16. All animal experiments were conducted in compliance with animal ethics regulations according to the Seoul National University Bundang Hospital IACUC Animal Experiment Plan (BA-2112-333-009-01).

Specifically, the experiment was conducted as follows: 4T1 murine triple-negative breast cancer cells (6×10⁵ cells in the right hind leg or 1×10⁵ cells in the left flank) were injected into the hind limb and flank of 6-week-old immune-competent BALB/c mice. Seven days after injection, the tumor was confirmed to have grown steadily, and the experimental group was divided into 7 groups, with five mice assigned to each group: ① Control group, ② Radiation treatment (RT) group, ③ IVAM-administered group, ④ IVAM-administered group + radiation combination treatment group, ⑤ a-PD-L1-administered group, ⑥ IVAM-administered group + a-PD-L1-administered group, ⑦ IVAM-administered group + a-PD-L1-administered group + radiation triple combination group. Here, each chemotherapy was performed as follows:
1) IVAM: ① 4T1 cells irradiated with 5, 20, 50, and 100 Gy were cultured for a week, and then the cell supernatant (with 1x10⁵ dead cells/injection) was collected at a ratio of 1:1:1:1, and 200 µl of the cell supernatant was intravenously injected once (on Day 10).
2) Radiation treatment (RT): Using X-RAD320 equipment from Precision X-ray Co., 8 Gy was administered two times (Days 11 and 12), and finally a total of 16 Gy was administered. Radiation was administered only to the right hind limb of the mice.
3) a-PD-L1: 100 µl was administered intraperitoneally four times (Day 11, 14, 17, and 20) at a concentration of 5 mg/kg.

The weight of mice in each group was measured and recorded every Monday, Wednesday, and Friday. All mice were euthanized on the 50th day after tumor implantation, and the survival rate of mice in each group for 50 days was measured. Statistical analysis was performed using one-way ANOVA (Tukey's multiple comparison tests) and unpaired two-tailed Student's t-tests of PRISM statistical analysis and graphing software (GraphPad 8).

### 5-2. Confirmation of long-term survival rate and toxicity according to IVAM, a-PD-L1, and radiation combination treatment

To determine the long-term survival rate and side effects of the IVAM therapy, a-PD-L1, and radiation combination treatment according to the present invention, the survival rate and body weight changes of mice were determined over time during the treatment course.

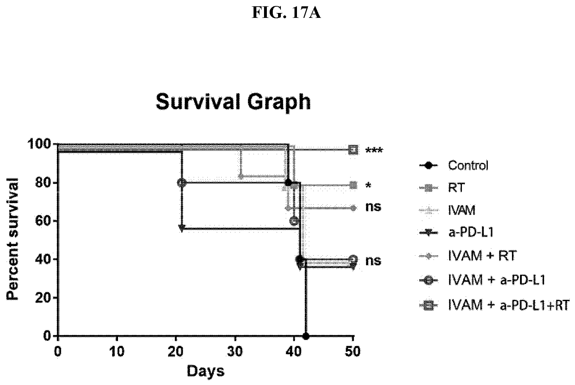

As a result of measuring the survival rates of the mice for 50 days, the survival rate decreased to 0% after 40 days in the case of the control group, whereas the survival rate was maintained at 100% even after 50 days (p<0.001) in the case of the IVAM, a-PD-L1, and radiation combination treatment. In the case of the radiation monotreatment group, it was also found that the survival rate was maintained at over 80% after 50 days (p<0.05) (FIG. 17A).

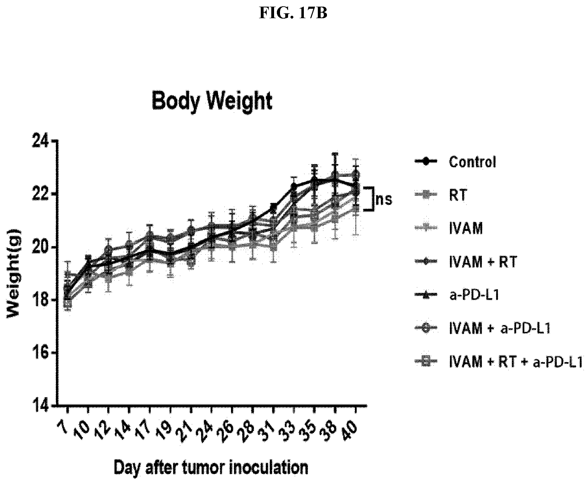

In addition, as a result of measuring the weight changes of the mice for 50 days, it was confirmed that there was no significant weight loss in any group treated with IVAM, a-PD-L1, and/or radiation compared to the control group (FIG. 17B).

The results suggest that the combined use of the IVAM, cancer immunotherapy drug, and radiation of the present invention can exert excellent anticancer and abscopal effects without causing side effects such as toxicity to the subject even for a long time.

### Example 6. Confirmation of anticancer effect and toxicity by multiple IVAM administrations

In this example, the anticancer effect of multiple IVAM administrations was confirmed through tumor size and IVIS imaging.

### 6-1. Experimental method

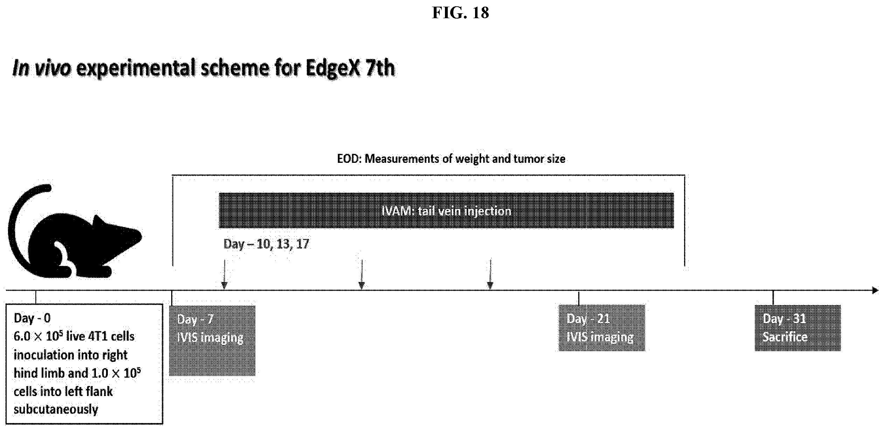

The animal experiment schedule for the anticancer effect and toxicity confirmation experiment through multiple IVAM administrations according to this example is shown in FIG. 18. All animal experiments were conducted in compliance with animal ethics regulations according to the Seoul National University Bundang Hospital IACUC Animal Experiment Plan (BA-2112-333-009-01). 4T1 murine triple-negative breast cancer cells (6×10⁵ cells in the right hind leg or 1×10⁵ cells in the left flank) were injected into the hind limb and flank of 6-week-old immune-competent BALB/c mice. Seven days after injection, the tumor was confirmed to have grown steadily, and experimental groups were assigned. The experimental groups were divided into a total of 4 groups, and 5 mice were assigned to each group: Control group; IVAM 1-dose group (IVAM-1); IVAM 2-dose group (IVAM-2); and IVAM 3-dose group (IVAM-3).

Specifically, IVAM administration was performed by seeding 4T1 cells in 60 pi dishes at 2.5 x 10⁵ cells/dish, irradiating them with 5, 20, 50, and 100 Gy each the next day, and culturing them for a week. The cell supernatants (with 1.5 x 10⁵ dead cells/injection) were collected in a ratio of 1:1:1:1, and 200 µl each was administered intravenously three times (on Days 10, 13, and 17).

The weight of mice in each group was measured and recorded every Monday, Wednesday, and Friday. On the 7th and 21st days after tumor implantation, the tumor growth inhibition effect, and the abscopal effect by radiation were compared and analyzed using IVIS imaging. All mice were euthanized on the 31st day after tumor implantation, and statistical analysis was performed using one-way ANOVA (Tukey's multiple comparison tests) and unpaired two-tailed Student's t-tests of PRISM statistical analysis and graphing software (GraphPad 8).

### 6-2. Confirmation of tumor growth inhibition effect and toxicity according to multiple IVAM administrations

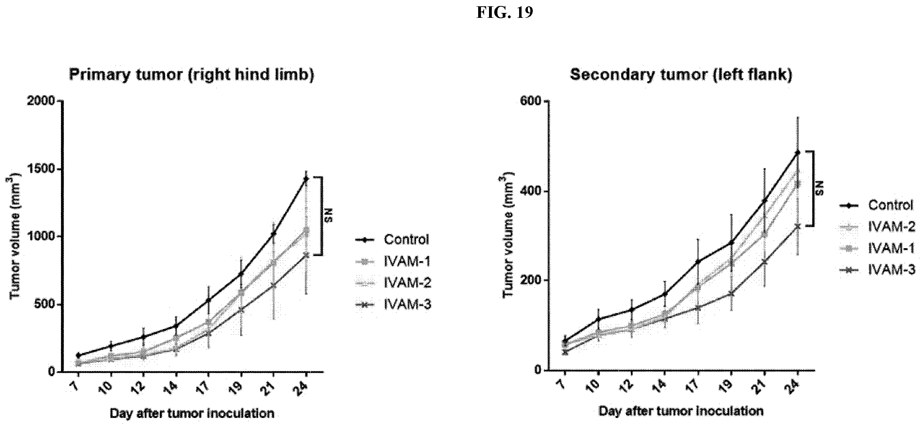

After tumor inoculation, tumor growth was observed for 24 days. As a result, the growth of the tumor (primary tumor) in the right hind limb that was irradiated with radiation and of the tumor (secondary tumor) in the left flank that was not irradiated with radiation was delayed in the IVAM-administered group compared to the control group, and was further delayed in the 3-dose group compared to the IVAM 1-dose and 2-dose groups, but it was not statistically significant (FIG. 19).

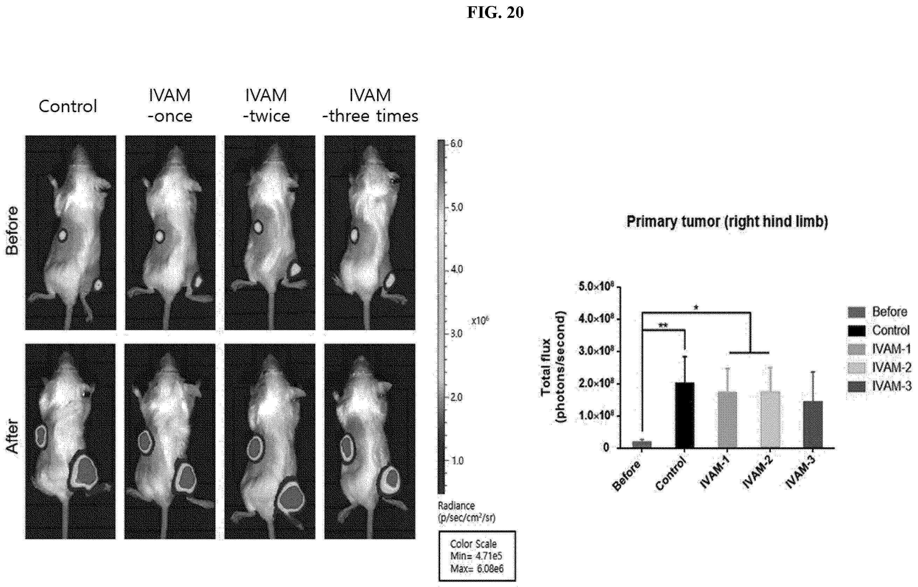

In addition, IVIS imaging using the luciferase system was used to compare and analyze the results on the 7th and 21st days after tumor implantation. As a result of measuring the size of the tumor directly, the tumor suppression effect was observed in the tumor (primary tumor) of the right hind limb irradiated with radiation in the IVAM-administered group, compared to the control group, and a higher suppression effect was observed in the 3-dose group compared to the IVAM 1-dose and 2-dose groups, similarly to the results confirmed above (FIG. 20).

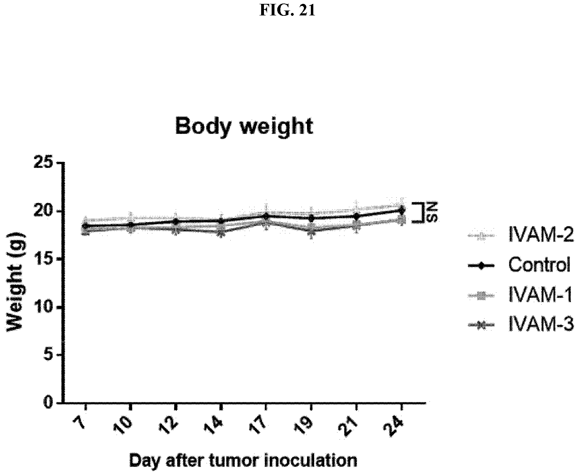

In addition, as a result of measuring the weight change of mice for 24 days, it was confirmed that there was no significant weight loss in all of the groups treated with IVAM once, twice, and three times compared to the control group (FIG. 21).

This result suggests that, even when the IVAM of the present invention is administered three times, anticancer effect and abscopal effect can be exhibited without causing side effects such as toxicity to the subject.

Through the above examples, the present inventors confirmed that when tumor tissue or cancer cells isolated from a cancer patient are treated with radiation and/or immunological treatment to suppress the activity of cancer cells or kill them, and then administered to a cancer patient, the immune system of the body can activate the immune function against cancer, can effectively suppress the progression or growth of primary cancer, and can also exert a tumor-killing function even against other tumors in the body. In particular, it was confirmed that, when radiation therapy was combined while re-administrating tumor cells that have been suppressed or killed by radiation irradiation (IVAM), not only the anticancer effect on primary tumors was further enhanced, but also the abscopal effect was exerted to suppress the growth of secondary tumors. Furthermore, the triple combination therapy that performs radiation therapy and immune checkpoint inhibitor treatment together with IVAM is expected to have a significant anticancer effect, and it is also expected that the activation function of immune cells will be achieved. Therefore, the composition and treatment method according to the present invention are expected to be used in the treatment of various cancers because they can enhance the body's immune function against tumors through relatively simple treatment and further enhance the anticancer effect of other chemotherapy.

Other objectives, features and advantages of the present invention will become apparent from the following detailed description. However, it should be understood that the detailed descriptions are provided for illustrative purposes only, and that various changes and modifications from the following detailed description within the spirit and scope of the present invention will become apparent to those skilled in the art.

### [Industrial Applicability]

The composition according to the present invention has tumor growth inhibition effect, anticancer immune function enhancement effect, and abscopal effect and thus is expected to be useful for preventing or treating cancer, thereby having industrial applicability.

## Claims

1. A pharmaceutical composition for preventing or treating cancer, comprising tumor tissue or cancer cells isolated from a cancer patient and applied with one or more treatments selected from the group consisting of (a) and (b) below, as an active ingredient:
(a) radiation irradiation; and
(b) a cancer immunotherapy drug.

2. The pharmaceutical composition according to claim 1, wherein the tumor tissue or the cancer cells satisfy one or more features selected from the group consisting of i) to iii) below by the treatment:
i) activity of cancer cells is weakened;
ii) activity of cancer cells is stopped; and
iii) cancer cells are killed.

3. The pharmaceutical composition according to claim 1, wherein the composition comprises autologous tumor tissue or cancer cells of the cancer patient and is administered to the cancer patient, or is administered to a cancer patient who is allogeneic with the cancer patient.

4. The pharmaceutical composition according to claim 1, wherein the composition is administered to a subject who does not have cancer to prevent cancer.

5. The pharmaceutical composition according to claim 1, wherein, when the treatment is (a) radiation irradiation, the radiation satisfies one or more features selected from the group consisting of i) and ii) below:
i) the radiation is one or more selected from the group consisting of γ-rays, x-rays, ultraviolet rays, laser rays, and infrared rays; and
ii) an irradiation dose of the radiation is 1 to 500 Gy.

6. The pharmaceutical composition according to claim 1, wherein the cancer immunotherapy drug is one or more selected from the group consisting of an immune checkpoint inhibitor, a co-stimulatory molecule agent, a cytokine therapeutic agent, a CAR-T cell therapeutic agent, and an autologous CD8+ T immune cell therapeutic agent.

7. The pharmaceutical composition according to claim 6, wherein the immune checkpoint inhibitor is one or more selected from the group consisting of a PD-L1 inhibitor, a PD-1 inhibitor, a CTLA-4 inhibitor, a LAG3 inhibitor, a TIM3 inhibitor, a 4-1BB inhibitor, a LAG-3 inhibitor, a B7-H4 inhibitor, a HVEM inhibitor, a TIM4 inhibitor, a GAL9 inhibitor, a VISTA inhibitor, a KIR inhibitor, a TIGIT inhibitor, and a BTLA inhibitor.

8. The pharmaceutical composition according to claim 1, wherein the composition satisfies one or more features selected from the group consisting of i) to iv) below when administered to a cancer patient:
i) exerts an abscopal effect;
ii) inhibits cancer metastasis;
iii) reduces tumor load; and
iv) inhibits proliferation of cancer cells.

9. The pharmaceutical composition according to claim 1, wherein the composition is intended for single or multiple administrations.

10. The pharmaceutical composition according to claim 1, wherein the composition comprises two or more tumor tissues or cancer cells, wherein the two or more tumor tissues or cancer cells are each subjected to same treatment or different treatments.

11. The pharmaceutical composition according to claim 10, wherein the composition is a mixture of two or more tumor tissues or cancer cells.

12. The pharmaceutical composition according to claim 10, wherein the composition is prepared by respectively formulating the two or more tumor tissues or cancer cells and is administered simultaneously, separately, or sequentially.

13. The pharmaceutical composition according to claim 1, wherein the composition is used in combination with anticancer therapy.

14. The pharmaceutical composition according to claim 13, wherein the anticancer therapy is one or more selected from the group consisting of radiation therapy, chemotherapy, targeted anticancer therapy, and cancer immunotherapy.

15. The pharmaceutical composition according to claim 13, wherein the composition is used in combination with radiation therapy and cancer immunotherapy.

16. The pharmaceutical composition according to claim 13, wherein the composition is administered simultaneously, separately, or sequentially with the anticancer therapy.

17. The pharmaceutical composition according to claim 14, wherein the targeted anticancer agent is one or more selected from the group consisting of a tyrosine kinase inhibitor, a PARP inhibitor, an angiogenesis inhibitor, and a CDK4/6 inhibitor.

18. The pharmaceutical composition according to claim 14, wherein the cancer immunotherapy drug is one or more selected from the group consisting of an immune checkpoint inhibitor, a co-stimulatory molecule agent, a cytokine therapeutic agent, a CAR-T cell therapeutic agent, and an autologous CD8⁺ T immune cell therapeutic agent.

19. The pharmaceutical composition according to claim 18, wherein the immune checkpoint inhibitor is one or more selected from the group consisting of a PD-L1 inhibitor, a PD-1 inhibitor, a CTLA-4 inhibitor, a LAG3 inhibitor, a TIM3 inhibitor, a 4-1BB inhibitor, a LAG-3 inhibitor, a B7-H4 inhibitor, a HVEM inhibitor, a TIM4 inhibitor, a GAL9 inhibitor, a VISTA inhibitor, a KIR inhibitor, a TIGIT inhibitor, and a BTLA inhibitor.

20. The pharmaceutical composition according to claim 1, wherein the tumor tissue is milled and diluted or dissolved in a solution.

21. The pharmaceutical composition according to claim 20, wherein the composition comprises two or more tumor tissues, wherein the two or more tumor tissues have identical or different milling particle sizes.

22. The pharmaceutical composition according to claim 1, wherein the cancer is one or more selected from the group consisting of blood cancer and solid cancer.

23. The pharmaceutical composition according to claim 1, wherein the cancer is one or more selected from the group consisting of breast cancer, colorectal cancer, lung cancer, head and neck cancer, small cell lung cancer, stomach cancer, liver cancer, blood cancer, bone cancer, pancreatic cancer, skin cancer, head cancer, neck cancer, cutaneous melanoma, intraocular melanoma, uterine cancer, ovarian cancer, rectal cancer, anal cancer, colon cancer, fallopian tube carcinoma, endometrial carcinoma, cervical cancer, vaginal cancer, vulvar carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocyte lymphoma, bladder cancer, kidney cancer, ureteral cancer, renal cell carcinoma, renal pelvic carcinoma, CNS tumors, primary CNS lymphoma, spinal cord tumors, brainstem glioma, and pituitary adenoma.

24. A kit for preventing or treating cancer, comprising the pharmaceutical composition of claim 1.

25. A method of preparing the composition according to claim 1, the method comprising:
(S1) isolating tumor tissue or cancer cells from a cancer patient; and
(S2) applying one or more treatments selected from the group consisting of (a) and (b) below to the isolated tumor tissue or cancer cells,
(a) radiation irradiation; and
(b) a cancer immunotherapy drug.

26. The method according to claim 25, wherein the method further comprises milling the tumor tissue and diluting or dissolving it in a solution after the step (S1).

27. The method according to claim 25, wherein the composition comprises autologous tumor tissue or cancer cells of the cancer patient, and is administered to the cancer patient or to a cancer patient allogeneic with the cancer patient.

28. The method according to claim 25, wherein the composition is administered to a subject who does not have cancer to prevent cancer.

29. An anticancer vaccine composition, comprising tumor tissue or cancer cells isolated from a cancer patient and applied with one or more treatments selected from the group consisting of (a) and (b) below as an active ingredient:
(a) radiation irradiation; and
(b) a cancer immunotherapy drug.

30. The anticancer vaccine composition according to claim 29, wherein the vaccine composition is administered to a subject, who does not have cancer or who has been cured of cancer, to prevent cancer.

31. A method of preventing or treating cancer, the method comprising administering a composition, which comprises tumor tissue or cancer cells isolated from a cancer patient and applied with one or more treatments selected from the group consisting of (a) and (b) below as an active ingredient, to a subject in need thereof:
(a) radiation irradiation; and
(b) a cancer immunotherapy drug.

32. Use of a composition comprising tumor tissue or cancer cells isolated from a cancer patient and applied with one or more treatments selected from the group consisting of (a) and (b) below as an active ingredient for preventing and treating cancer:
(a) radiation irradiation; and
(b) a cancer immunotherapy drug.

33. Use of tumor tissue or cancer cells isolated from a cancer patient and applied with one or more treatments selected from the group consisting of (a) and (b) below, for preparing a preparation for prevention or treatment of cancer:
(a) radiation irradiation; and
(b) a cancer immunotherapy drug.
